(19) Europäisches Patentamt
European Patent Office
Office européen des brevets

(11) Veröffentlichungsnummer : **0 466 639 B1**

# (12) EUROPÄISCHE PATENTSCHRIFT

(45) Veröffentlichungstag der Patentschrift :
**10.08.94 Patentblatt 94/32**

(51) Int. Cl.[5] : **C10M 159/18,** C10M 135/16, C10M 141/10, C10M 163/00, C07F 1/00

(21) Anmeldenummer : **91810474.6**

(22) Anmeldetag : **19.06.91**

## (54) Schmierstoffzusammensetzung.

(30) Priorität : **28.06.90 CH 2169/90**

(43) Veröffentlichungstag der Anmeldung :
**15.01.92 Patentblatt 92/03**

(45) Bekanntmachung des Hinweises auf die Patenterteilung :
**10.08.94 Patentblatt 94/32**

(84) Benannte Vertragsstaaten :
**BE DE FR GB IT**

(56) Entgegenhaltungen :
**EP-A- 0 024 146**
**EP-A- 0 072 349**
**EP-A- 0 376 889**
**WO-A-86/06378**
**FR-A- 2 511 699**
**US-A- 4 824 601**
**CHEMISCHE BERICHTE. Bd. 120, 7. Juli 1987, WEINHEIM DE Seiten 1251 - 1253;KÖNIG ET AL.: 'SYMETRISCHE 3,3,3',3'-TETRAALKYL1,1'-ALKANDIOYLBIS(THIOHARNSTOFFE) ALS NEUE CHELATLIGANDEN '**

(56) Entgegenhaltungen :
**JOURNAL FÜR PRAKTISCHE CHEMIE Bd. 317, Nr. 5, 1975, LEIPZIG-BRD Seiten 829 -839; BEYER ET AL.: 'SYNTHESE UND CHARAKTERISIERUNG NEUARTIGERU-¨BERGANGSMETALLCHELATE VON 1,1-DIALKYL-3-BENZOYL-THIOHARNSTOFFEN '**
**JOURNAL OF MEDICINAL CHEMISTRY. Bd. 15, Nr. 3, 1972, WASHINGTON US Seiten 315 -320; OLIVER ET AL.: 'INSECT CHEMOSTERILANTS. 11. SUBSTITUTED 3,5-DIAMINO-1,2,4-DITHIAZOLIUM SALTS AND RELATED COMPOUNDS '**
**CHEMICAL ABSTRACTS, vol. 87, no. 4, 25. Juli 1977, Columbus, Ohio, US; abstractno. 33049X, GEETHARANI ET AL. 'MONOTHIOBIURET COMPLEXES OF SOME BIVALENT METAL-CHLORIDES ' Seite 685 ;Spalte 2 ;**

(73) Patentinhaber : **CIBA-GEIGY AG**
**Klybeckstrasse 141**
**CH-4002 Basel (CH)**

(72) Erfinder : **Camenzind, Hugo, Dr.**
**Herzogstrasse 25**
**CH-3014 Bern (CH)**
Erfinder : **Schumacher, Rolf, Dr.**
**Chemin de la Combettaz 40**
**CH-1723 Marly (CH)**

EP 0 466 639 B1

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft neue Zusammensetzungen, enthaltend einen Schmierstoff oder eine Hydraulikflüssigkeit und als Antioxidans mindestens einen Kupferkomplex eines N-acylierten Thiohamstoffes, die Verwendung dieser Kupferkomplexe sowie ein Verfahren zur Stabilisierung von Schmierstoffen oder Hydraulikflüssigkeiten gegen oxidativen oder thermischen Abbau.

Es ist bekannt, Schmierstoffen, wie Mineralölen oder synthetischen und halbsynthetischen Oelen, Zusatzstoffe zur Verbesserung der Gebrauchseigenschaften zuzusetzen.

Von grosser Bedeutung sind Zusatzstoffe, die den oxidativen Abbau der Schmierstoffe unterbinden und eine hohe Lager- und Wirkungsstabilität gewährleisten.

Insbesondere das thermooxidative Anforderungsprofil modemer Motorenöle hat sich infolge neuer Motorenkonstruktionen im Bereich der Verbrennungskraftmaschinen mit Eigen- oder Fremdzündung geändert. So bilden sich beispielsweise bei Motoren mit Fremdzündung bei der heutigen Motorenauslegung und Betriebsweise vermehrt Stick-oxide, die wiederum als Durchblasgase ("blow-by"-Gase) ins Kurbelgehäuse gelangen.

Weiters übernimmt das Schmieröl im oberen Kolbenring- und Zylinderbereich die Feinabdichtung zum Verbrennungsraum. Hier kann es zur Kontamination mit hochsiedenden Kraftstoffkomponenten kommen. Diese vorgegebenen Bedingungen werden durch die Anwesenheit von $NO_x$ verschärft.

Die Durchblasgase, die zunehmend höhere $NO_x$-Anteile aufweisen, bewirken nun eine grössere Oxidationsanfälligkeit des Schmieröles und es bilden sich "Schlammkeime", die letztlich zu unerwünschten Schlammablagerungen führen, die als "Schwarzschlamm" bekannt geworden sind.

Es ist davon auszugehen, dass es sich dabei um eine $NO_x$-initiierte Autooxidation des Schmieröles handelt.

Es hat nicht an Versuchen gefehlt, Schmieröle durch Zusätze von Antioxidantien zu verbessern.

Kupferverbindungen als Antioxidantien in Schmierstoffen sind bekannt.
So werden in der US-A 3,351,647 phosphor- und stickstoffhaltige Komplexe von Metallen, neben anderen auch von Kupfer, mit antioxidativer Wirksamkeit in Schmierstoffen beschrieben. Diese Komplexe zeigen einen synergistischen Effekt mit Phenyl-β-naphthylamin. Aus der US-A 3,634,238 ist weiter eine Kombination von Metallen oder Metallcarbonsäuresalzen, u.a. auch von Kupfer, mit aromatischen Aminen mit synergistischer, antioxidativer Wirkung in organischem Material, insbesondere Schmierstoffen, bekannt.

Ferner wird in der EP-A 24 146 eine Kombination von Dispergator, Viskositätsindex-Verbesserer, Zinkdithiophosphat und Kupferverbindungen im ppm-Bereich als antioxidatives System in Schmierstoffen beschrieben.

Ferner beschreibt die US-A 4,828,733 Kupfersalze von sterisch gehinderten Phenol-carbonsäuren als Antioxidantien in Schmierstoffen und erwähnt einen synergistischen Effekt bei der Verwendung mit anderen Antioxidantien wie z.B. phenolischen oder Aryl-aminantioxidantien.

Es wurde nun überraschend gefunden, dass sich Kupferkomplexe von 1, 1 -disubstituierten 3-Acylthiohamstoffen ausgezeichnet als Antioxidantien für Schmierstoffe eignen.

1,1-Dialkyl-3-benzoylthioharnstoffe werden von L. Beyer et al. in Journal für praktische Chemie, Band 317, Heft 5, 1975, S. 829-839, als ausgezeichnete Chelatbildner für Uebergangsmetallionen wie z.B. Kobalt, Kupfer, Nickel und Palladium beschrieben.

Weiter sind symmetrische 3,3,3′,3′-Tetraalkyl-1, 1-alkandioylbis(thioharnstoffe) aus K.-H. König et al., Chemische Berichte 120, S. 1251-1253, (1987) als Chelatliganden, die sich zur Abtrennung und extraktiven Anreicherung von Platinmetallen eignen, bekannt.

Die vorliegende Erfindung betrifft eine Zusammensetzung enthaltend

a) einen Schmierstoff oder eine Hydraulikflüssigkeit und

b) mindestens eine Verbindung der allgemeinen Formel I

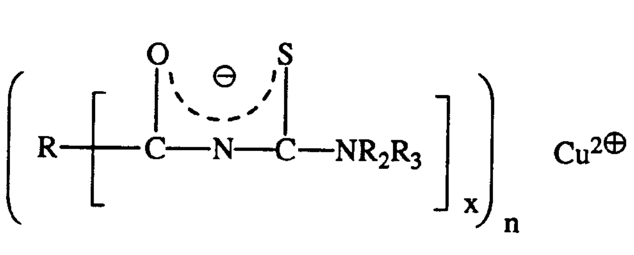

(I),

wobei x=1 oder 2 ist und, wenn x=1 ist, n=2 ist und R die Bedeutung von $R_1$ hat, oder, wenn x=2 ist, n=1 ist und R die Bedeutung von $R_4$ hat, und $R_1$ Alkyl mit 1-25 C-Atomen, eine unsubstituierte oder $C_1$-$C_8$-alkylsubstituierte Cycloalkylgruppe mit 5-12 C-Atomen, Alkenyl mit 2-18 C-Atomen, Phenyl, Naphthyl, $C_7$-$C_{18}$-Phenyl-

alkyl, $C_7$-$C_{18}$-Alkylphenyl oder -$NR_5R_6$ bedeutet, und $R_2$ und $R_3$ gleich oder verschieden sind und Alkyl mit 1-18 C-Atomen, Phenyl, Naphthyl, $C_7$-$C_{18}$-Phenylalkyl oder $C_7$-$C_{18}$-Alkylphenyl bedeuten, oder $R_2$ und $R_3$, zusammen mit dem sie verbindenden N-Atom, einen 5- bis 7-gliedrigen heterozyklischen Ring bilden, der gegebenenfalls zusätzlich ein N- oder O-Atom enthalten kann, $R_4$ Alkylen mit 1-12 C-Atomen, das gegebenenfalls durch mindestens eine -O-Gruppe unterbrochen ist, darstellt oder Alkenylen mit 2-12 C-Atomen ist, das gegebenenfalls durch mindestens eine -O-Gruppe unterbrochen ist, und $R_5$ und $R_5$ die gleichen Bedeutungsmöglichkeiten wie $R_2$ und $R_3$ haben.

In der Struktur der Formel I ist die negative Ladung nicht fixiert. Die Formel beinhaltet somit die verschiedenen möglichen mesomeren Grenzstrukturen, wie sie z.B. durch folgende Formeln dargestellt werden können

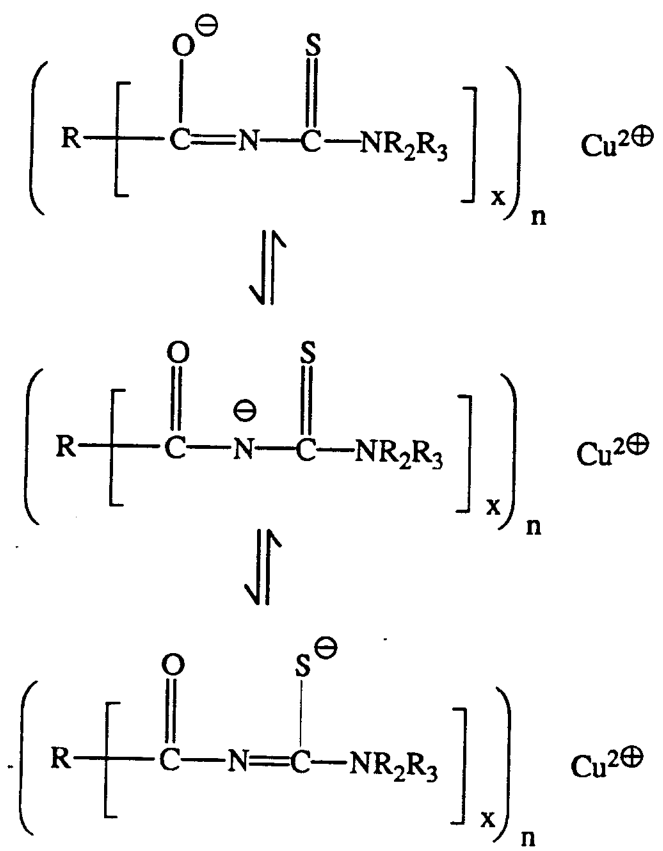

Stellt $R_1$ einen Alkylrest mit 1 bis 25 C-Atomen dar, so kann die Alkylgruppe geradkettig oder verzweigt sein und kann beispielsweise bedeuten: Methyl, Ethyl, Propyl, Isopropyl, n-Butyl, Isobutyl, t-Butyl, Pentyl, Isopentyl, Hexyl, Heptyl, 3-Heptyl, Octyl, 2-Ethylhexyl, Nonyl, Decyl, Undecyl, Dodecyl, Tridecyl, Tetradecyl, Pentadecyl, Hexadecyl, Heptadecyl, Octadecyl, Eicosyl, Henicosyl, Docosyl, 2-Ethylbutyl, 1-Methylpentyl, 1,3-Di-methylbutyl, 1,1,3,3-Tetramethylbutyl, 1-Methylhexyl, Isoheptyl, 1-Methylheptyl, 1,1,3-Trimethylhexyl, 1,1,3,3-Tetramethylhexyl oder 1-Methylundecyl. Bevorzugt stellt $R_1$ einen Alkylrest mit 1-18 C-Atomen dar.

Alkylreste können als geradkettige oder verzweigte Reste vorliegen, wobei insbesondere die verzweigtkettigen Reste als Gemische ihrer Isomeren vorliegen können.

$R_1$ kann auch Alkenyl mit 2 bis 18 C-Atomen bedeuten. Beispiele dafür sind Vinyl, Allyl, 2-Methallyl, Butenyl, wie z.B. 2-Butenyl, Hexenyl, wie z.B. 2-Hexenyl, Decenyl, Undecenyl, wie z.B. 10-Undecenyl, Heptadecenyl oder Oleyl.

Beispiele für die obengenannten unsubstituierten oder $C_1$-$C_8$-alkylsubstituierten Cyclo-alkylgruppen mit 5-12 C-Atomen sind Cyclopentyl, Cyclohexyl, Cyclooctyl, Cyclo-dodecyl, 2- oder 4-Methylcyclohexyl, Dimethylcyclohexyl, Trimethylcyclohexyl, t-Butyl-cyclohexyl, wobei Cyclohexyl besonders bevorzugt ist.

Stellen $R_1$, $R_2$ und $R_3$ $C_7$-$C_{18}$-Phenylalkyl dar, so handelt es sich beispielsweise um Benzyl, 1- oder 2-Phenylethyl, 3-Phenylpropyl, α,α-Dimethylbenzyl, 2-Phenylisopropyl, 2-Phenylhexyl oder Benzhydryl, vorzugsweise jedoch um Benzyl.

$R_1$, $R_2$ und $R_3$ als $C_7$-$C_{18}$-Alkylphenyl können lineare oder verzweigte Alkylgruppen enthalten, wobei 1-3, insbesondere 1 oder 2 Alkylgruppen vorhanden sind. Beispiele sind Tolyl, Ethylphenyl, Isopropylphenyl,

tert.Butylphenyl, sec.Pentylphenyl, n-Hexylphenyl, tert.Octylphenyl, iso-Nonylphenyl oder n-Dodecylphenyl.

$R_2$ und $R_3$ können gleich oder verschieden sein und sind bevorzugt gleich. Auch $R_5$ und $R_5$ sind vorzugsweise gleich. Weiterhin bevorzugt sind Verbindungen der Formel I, worin $-NR_2R_3$ und $-NR_5R_6$ gleich sind.

Stellen $R_2$ und $R_3$ einen Alkylrest mit 1 bis 18 C-Atomen dar, so können beispielsweise Methyl, Ethyl, n-Propyl, i-Propyl, n-Butyl, i-Butyl, n-Pentyl, n-Hexyl, n-Heptyl, n-Octyl, 2-Ethylhexyl, Nonyl, Decyl, Undecyl, Dodecyl, Heptadecyl oder Octadecyl genannt werden.

Beispiele für $-NR_2R_3$ bzw. $-NR_5R_6$ sind die N,N-Di-i-Butyl-, N,N-Di-n-Hexyl-, N,N-Di-t-Octyl-, N,N-Di-2-Ethylhexyl-, N,N-Didodecyl-, N-Methyl-N-Phenyl- oder N-Dodecyl-N-Phenylreste.

Stellen $R_2$ und $R_3$, zusammen mit dem N-Atom, an das sie gebunden sind, einen 5- bis 7-gliedrigen heterozyklischen Ring dar, der gegebenenfalls zusätzlich ein weiteres N- oder ein O-Atom enthält, handelt es sich vorzugsweise um gesättigte Ringe, insbesondere um 6-gliedrige. Beispiele dafür sind der Piperidin-, Hexamethylenimin-, Piperazin- oder Morpholinring.

$R_4$ hat u.a. die Bedeutung von Alkylen mit 1 bis 12 C-Atomen, wozu die Beispiele Methylen, Ethylen, Trimethylen, 2,2-Dimethyl- 1,3-propandiyl, Tetramethylen, Pentamethylen, Hexamethylen, Heptamethylen, Octamethylen, Decamethylen oder Dodecamethylen gehören. Trimethylen, Tetramethylen, Hexamethylen und Octamethylen werden bevorzugt.

Beispiele für $R_4$ als Alkylen, das durch Sauerstoffatome, vorzugsweise 1 oder 2, unterbrochen ist, sind 3-Oxapentan- 1,5-diyl; 3,6-Dioxaoctan- 1,8-diyl; 2-Oxapropan- 1,3-diyl; 2,7-Dioxaoctan- 1,8-diyl oder 2,6-Dioxa-4,4-dimethyl- 1,7-heptandiyl.

Stellt $R_4$ Alkenylen mit 2- 12 C-Atomen dar, kann dieses eine oder mehrere, bevorzugt jedoch eine, Doppelbindungen enthalten und geradkettig oder verzweigt sein. Beispiele für solche Alkenylreste sind: Vinylen, Alkylen, 2-Methalkylen, 2-Hexenylen, 2-Methyl-3-butenylen, 4-Propyl-2-pentenylen, 2-Decenylen oder Dodecenylen.

Stellt $R_4$ Alkenylen dar, das durch 1 oder 2 Sauerstoffatome unterbrochen ist, handelt es sich beispielsweise um 3-Oxa-5-heptenylen, 2,7-Dioxa-4-octenylen, 3,8-Dioxa-5-decenylen oder 3-Oxa-5,8-undecadienylen.

Bevorzugt sind Zusammensetzungen, die mindestens eine Verbindung der Formel I enthalten, worin $R_1$ Alkyl mit 1-18 C-Atomen, eine unsubstituierte Cycloalkylgruppe mit 5-8 C-Atomen, Phenyl, $C_7$-$C_{12}$-Alkylphenyl, $-NR_5R_6$ oder $C_7$-$C_{12}$-Phenylalkyl bedeutet, $R_2$ und $R_3$ bzw. $R_5$ und $R_5$ gleich oder verschieden sind und jeweils Alkyl mit 1-18 C-Atomen oder Phenyl bedeuten, oder $R_2$ und $R_3$ bzw. $R_5$ und $R_5$, zusammen mit dem sie verbindenden N-Atom, eine Piperidino-, Hexamethylenimino-, Piperazino- oder Morpholino-gruppe bilden, und $R_4$ Alkylen mit 1-12 C-Atomen oder Alkenylen mit 2-12 C-Atomen darstellt.

Es ist im Umfange der vorliegenden Erfindung, dass in den Zusammensetzungen auch mehrere Verbindungen der Formel I in beliebigem Gemisch untereinander, angewendet werden können.

Besonders bevorzugt sind Zusammensetzungen, die Verbindungen der Formel I enthalten, worin $R_1$ Alkyl mit 1-18 C-Atomen, Phenyl, $-NR_5R_6$ oder Benzyl, insbesondere jedoch Alkyl mit 6-18 C-Atomen, $-NR_5R_6$ oder Phenyl, darstellt, und $R_2$ und $R_3$ bzw. $R_5$ und $R_6$ gleich oder verschieden sind und Alkyl mit 1-18 C-Atomen, insbesondere jedoch Alkyl mit 1-12 C-Atomen, bedeuten und $R_4$ für Alkylen mit 2-10 C-Atomen steht.

Weiter sind Zusammensetzungen besonders bevorzugt, die mindestens eine Verbindung der Formel I enthalten, worin $R_2$ und $R_3$ unabhängig voneinander Alkyl mit 4-8 C-Atomen darstellen und $R_4$ Alkylen mit 1-10 C-Atomen oder Alkenylen mit 2- 10 C-Atomen, insbesondere jedoch Alkylen mit 3- 8 C-Atomen, bedeutet.

Ganz besonders bevorzugt sind Zusammensetzungen, die mindestens eine Verbindung der Formel I enthalten, worin $R_1$ Phenyl oder $-NR_5R_5$ darstellt und $R_2$ und $R_3$ bzw. $R_5$ und $R_5$ unabhängig voneinander jeweils Alkyl mit 1-8 C-Atomen bedeuten.

Ebenfalls ganz besonders bevorzugt sind Zusammensetzungen, die mindestens eine Verbindung der Formel I enthalten, worin, wenn $x = 1$ ist, $R_1$ Phenyl oder $-NR_5R_5$ bedeutet, und $R_2$ und $R_3$ bzw. $R_5$ und $R_6$ gleich sind und je Alkyl mit 1-12 C-Atomen darstellen, oder worin, wenn $x = 2$ ist, $R_4$ Alkylen mit 3-8 C-Atomen ist, und $R_2$ und $R_3$ je Alkyl mit 3-10 C-Atomen bedeuten.

Weiter ganz speziell bevorzugt sind Zusammensetzungen, die mindestens eine Verbindung der Formel I enthalten, worin, wenn $x=1$ ist, $R_1$ Phenyl bedeutet, und $R_2$ und $R_3$ gleich sind und je Ethyl, Propyl, n-Butyl, Isobutyl, Hexyl oder 2-Ethylhexyl darstellen, oder worin, wenn $x=2$ ist, $R_4$ Tetramethylen ist und $R_2$ und $R_3$ je 2-Ethylhexyl bedeuten.

Die Verbindungen der Formel I sind z.T. aus den nachfolgend zitierten Literaturstellen bekannt oder können beispielsweise nach den an sich bekannten Methoden hergestellt werden.

Solche Methoden werden in L. Beyer et al., Journal für praktische Chemie, Band 317, Heft 5, 1975, S. 829-939; H. Hartmann et al., Journal für praktische Chemie, Band 315, Heft 1, 1973, S. 144-148, und in K.-H. König et al., Chemische Berichte 120, S. 1251- 1253, (1987) beschrieben.

In Anlehnung an die an sich bekannten Methoden können die Liganden in den Verbindungen der Formel

I (Verbindungen der Formel I ohne Cu) nach folgenden allgemeinen Gleichungen hergestellt werden:

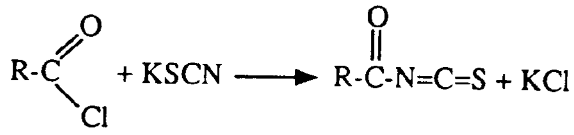

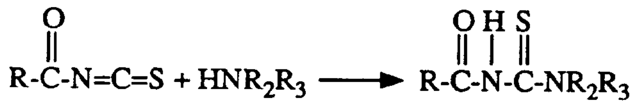

bzw.

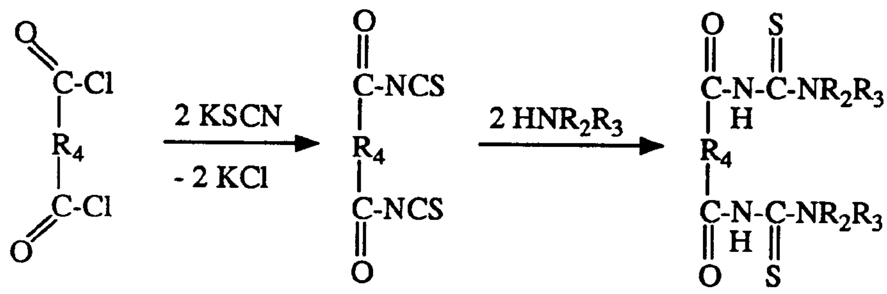


Die Liganden von Verbindungen der Formel I, in denen $R_1$ $-NR_5R_6$ bedeutet, können nach folgendem Reaktionsschema hergestellt werden:

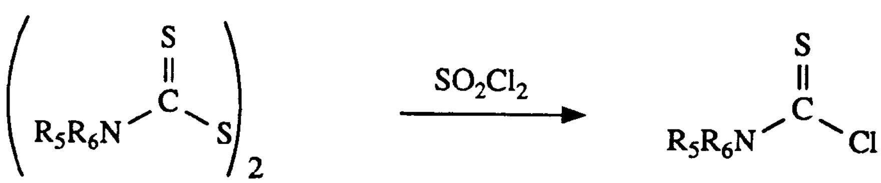


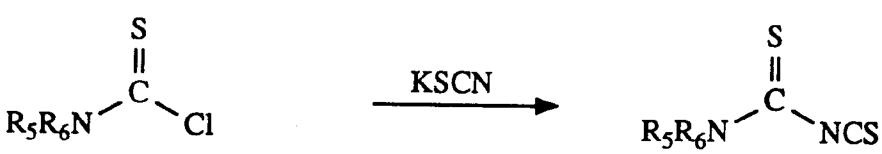


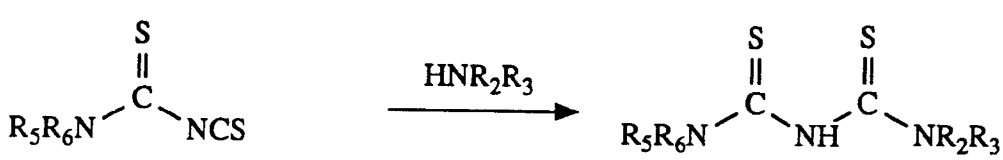


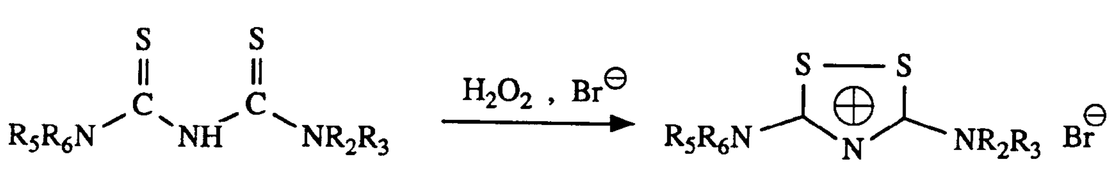

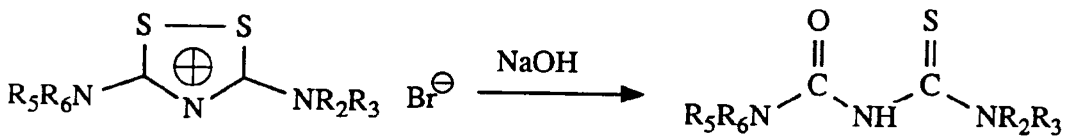


Siehe dazu auch A.N. Pudovik et al., Zh. Obshch. Khim., Band 58, Heft 7, 1988, S. 1489-1493; J.E. Oliver et al., J. Medicinal Chem., Band 15, Heft 3, 1972, S. 315-320.

Die Bezeichnungen $R_1$, $R_2$, $R_3$, $R_4$, $R_5$ und $R_6$ haben die weiter oben erläuterten Bedeutungen.

Die Ausgangsstoffe, beispielsweise ein Acylchlorid, ein Thiocyanat und das gewählte Amin werden beispielsweise in einem polaren aprotischen Lösungsmittel bei Temperaturen zwischen etwa 50°C und 70°C zur Umsetzung gebracht.

Geeignete Acylchloride lassen sich sinngemäss aus den oben genannten Bedeutungen für $R_1$ ableiten und dies sind beispielsweise Acetylchlorid, Butyrylchlorid, Oleolylchlorid, 3-Phenyl-propionylchlorid, Benzoylchlorid oder für $R_4$ Glutarsäuredichlorid, Adipinsäuredichlorid, Korksäuredichlorid, Sebacinsäuredichlorid usw.

Als Thiocyanate lassen sich beispielsweise Ammoniumthiocyanat, Natriumthiocyanat und bevorzugt Kaliumthiocyanat aufzählen.

Entsprechend den Bedeutungen für $R_2$ resp. $R_3$ können als Amine z.B. n-Butylamin, 2-Ethyl-hexylamin, Dihexylamin, Bis(2-Ethylhexyl)amin, Methylanilin, Piperidin, Hexamethylenimin, Morpholin, Pyrrolidin oder Piperazin genannt werden.

Die Aufzählung von Ausgangsprodukten ist als beispielhaft anzusehen. Die vollständigen Ausgangsprodukte ergeben sich sinngemäss und entsprechend aus allen Substituenten, die für R, $R_2$ und $R_3$ in Frage kommen.

Die Verbindungen der Formel I können leicht über die Komplexierung der SchwefelStickstoffliganden mit Kupfer(II)salzen bei erhöhter Temperatur, wie z.B. bei 50-60°C, in einem Lösungsmittel wie beispielsweise Methanol, Ethanol, Toluol oder Xylol hergestellt werden. Die Umsetzungen sind fast quantitativ, und die Komplexe lassen sich gut durch Kristallisation reinigen.

Die erfindungsgemässen Zusammensetzungen enthalten als weitere Komponente einen Schmierstoff oder eine Hydraulikflüssigkeit. Als Schmierstoffe können die an sich bekannten Produkte zur Anwendung gelangen.

Die gesuchten Eigenschaften der erfindungsgemässen Verbindungen kommen auch in den Hydraulikflüssigkeiten voll zum Tragen, wenn auch in diesem Falle der Asche- und Phosphorarmut oder -freiheit nicht die obenerwähnte Bedeutung zukommt.

Die in Frage kommenden Schmierstoffe und Hydraulikflüssigkeiten sind dem Fachmann geläufig und z.B. in Dieter Klamann "Schmierstoffe und verwandte Produkte", Verlag Chemie, Weinheim, 1982, in Schewe-Kobek, "Das Schmiermittel-Taschenbuch", Dr. Alfred Hüthig-Verlag, Heidelberg, 1974, oder in "Ullmanns Encyclopädie der technischen Chemie", Band 13, Seiten 85-94 (Verlag Chemie, Weinheim, 1977) beschrieben.

Beispiele hierfür sind Schmierstoffe und Hydraulikflüssigkeiten auf Basis von Mineralölen, synthetischen Oelen oder Mischungen mineralischer und synthetischer Oele, oder synthetischer Schmierstoffe oder Hydraulikflüssigkeiten, beispielsweise solche, die Carbonsäure-Esterderivate darstellen und bei Temperaturen von 200°C und höher verwendet werden.

Beispiele von synthetischen Schmierstoffen umfassen auch Schmierstoffe auf der Basis eines Diesters einer zweiwertigen Säure mit einem einwertigen Alkohol, wie z.B. Dioctylsebacat oder Dinonyladipat, eines Triesters von Trimethylolpropan mit einer einwertigen Säure oder mit einem Gemisch solcher Säuren, wie z.B. Trimethylolpropantripelargonat, Trimethylolpropan-tricaprylat oder Gemische davon, eines Tetraesters von Pentaerythrit mit einer einwertigen Säure oder mit einem Gemisch solcher Säure, wie z.B. Pentaerythrit-tetracaprylat, oder eines komplexen Esters von einwertigen und zweiwertigen Säuren mit mehrwertigen Alkoholen, wie z.B. ein komplexer Ester von Trimethylolpropan mit Capryl- und Sebacinsäure oder von einem Gemisch davon.

Besonders geeignet sind neben Mineralölen z.B. Poly-α-Olefine, Schmierstoffe auf Esterbasis, Phosphate, Glykole, Polyglykole und Polyalkylenglykole, sowie deren Mischungen mit Wasser.

Die Verbindungen der Formel I sind gut in Schmierstoffen und Hydraulikflüssigkeiten löslich und sind deshalb als Zusätze zu Schmierstoffen und Hydraulikflüssigkeiten besonders geeignet, und es ist auf ihre überraschend gute antioxidative Wirkung hinzuweisen.

Beispielsweise in Schmierstoffen für Verbrennungsmotoren, wie z.B. in Verbrennungsmotoren nach dem

Otto-Prinzip, vermögen die Verbindungen der Formel I ihre überragenden Eigenschaften zu entfalten. So wirken die Verbindungen der Formel I im Schmieröl antioxidativ, ohne jedoch nachteilig auf ein katalytisches Abgasreinigungssystem einzuwirken.

Die Verbindungen der Formel I wirken schon in sehr geringen Mengen als Additive in Schmierstoffen und Hydraulikflüssigkeiten. Sie werden den Schmierstoffen und Hydraulikflüssigkeiten zweckmässig in einer Menge von 0,01 bis 5 Gew.-%, vorzugsweise in einer Menge von 0,05 bis 3 Gew.-% und ganz besonders bevorzugt in einer Menge von 0,1 bis 2 Gew.-%, jeweils bezogen auf den Schmierstoff oder die Hydrauliflüssigkeit, beigemischt.

Eine ganz spezielle Wirksamkeit zeigen die Verbindungen der Formel I zusammen mit aromatischen Aminen oder/und Alkalidithiophosphaten.

Daher betrift die vorliegende Erfindung auch eine Zusammensetzung, enthaltend

a) einen Schmierstoff oder eine Hydraulikflüssigkeit,

b) mindestens eine Verbindung der Formel I

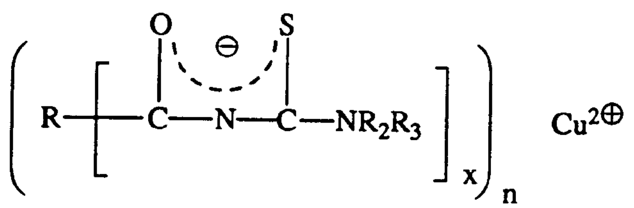

(I),

worin R, $R_2$ und $R_3$ die vorstehend angegebenen Bedeutungen haben, und

c) mindestens ein aromatisches Amin oder/und mindestens ein Alkalidithiophosphat.

Zweckmässig handelt es sich bei dem aromatischen Amin um eine Verbindung der Formeln II, III oder eine Mischung von solchen Verbindungen,

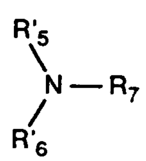

(II)

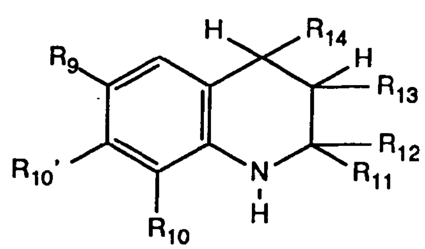

(III)

worin $R'_5$ $C_1$-$C_{18}$-Alkyl, $C_7$-$C_9$-Phenylalkyl, $C_5$-$C_{12}$-Cycloalkyl, Phenyl, $C_7$-$C_{18}$-Alkylphenyl, $C_7$-$C_{18}$-Alkoxyphenyl oder Naphthyl bedeutet,

$R'_6$ Phenyl, $C_7$-$C_{18}$-Alkylphenyl, $C_7$-$C_{18}$-Alkoxyphenyl oder Naphthyl bedeutet,

$R_7$ Wasserstoff, $C_1$-$C_{12}$-Alkyl, Benzyl, Ally, Methallyl, Phenyl oder eine Gruppe $-CH_2SR_8$ bedeutet, und

$R_8$ $C_4$-$C_{18}$-Alkyl, $-CH_2COO(C_4$-$C_{18}$-Alkyl) oder $-CH_2CH_2COO(C_4$-$C_{18}$-Alkyl) bedeutet, und

$R_9$ und $R_{10}$ unabhängig voneinander Wasserstoff, $C_1$-$C_{18}$-Alkyl oder Benzyl bedeuten, und

$R_{10'}$ Wasserstoff oder $C_1$-$C_{12}$-Alkyl bedeutet oder zusammen mit $R_{10}$ einen Butadiendiyl-Rest bildet, und

$R_{11}$ und $R_{12}$ unabhängig voneinander $C_1$-$C_{18}$-Alkyl, Phenyl oder Benzyl sind oder $R_{11}$ und $R_{12}$ zusammen mit dem Kohlenstoffatom, an welches sie gebunden sind, einen $C_5$-$C_{12}$-Spiro-Cycloalkylring bilden, und

$R_{13}$ Wasserstoff oder $C_1$-$C_{18}$-Alkyl und

$R_{14}$ $C_1$-$C_{18}$-Alkyl ist, oder

$R_{13}$ und $R_{14}$ zusammen mit den beiden Kohlenstoffatomen, an die sie gebunden sind, einen $C_5$-$C_{12}$-cycloaliphatischen Rest bedeuten enthalten sind.

Bevorzugte Verbindungen der Formel II sind die, worin

$R'_5$ $C_1$-$C_4$-Alkyl, $C_7$-$C_9$-Phenylalkyl, Cyclohexyl, Phenyl, $C_{10}$-$C_{18}$-Alkylphenyl oder Naphthyl bedeutet,

$R'_6$ $C_{10}$-$C_{18}$-Alkylphenyl oder Phenyl bedeutet,

$R_7$ Wasserstoff, $C_1$-$C_8$-Alkyl, Benzyl, Allyl oder eine Gruppe $-CH_2SR_8$ bedeutet,

$R_8$ $C_8$-$C_{18}$-Alkyl oder $-CH_2COO(C_8$-$C_{18}$-Alkyl) bedeutet, insbesondere jedoch die, worin $R'_5$ und $R'_6$ unabhängig voneinander Phenyl oder $C_{10}$-$C_{18}$-Alkylphenyl bedeuten und $R_7$ Wasserstoff ist.

Speziell bevorzugt als Verbindung der Formel II wird ein technisches Gemisch, erhalten durch Reaktion von Diphenylamin mit Diisobutylen, wie z.B. in der US-A 4,824,601 beschrieben, enthaltend

| | |
|---|---|
| 1 bis 5 Gew.-% | a) Diphenylamin |
| 8 bis 18 Gew.-% | b) 4-tert-Butyldiphenylamin |
| 21 bis 31 Gew.-% | c) einer oder mehrerer der Verbindungen |
| | i) 4-tert-Octyldiphenylamin |
| | ii) 4,4′-Di-tert-butyldiphenylamin |
| | iii) 2,4,4′-Tris-tert-butyldiphenylamin, |
| 20 bis 31 Gew.-% | d) einer oder mehrerer der Verbindungen |
| | i) 4-tert-Butyl-4′-tert-octyldiphenylamin |
| | ii) 2,2′- oder 2,4′-Di-tert-octyldiphenylamin |
| | iii) 2,4-Di-tert-butyl-4′-tert-octyldiphenylamin und |
| 15 bis 29 Gew.-% | e) der Verbindung |
| | i) 4,4′-Di-tert-octyldiphenylamin oder der Verbindungen |
| | i) 4,4′-Di-tert-octyldiphenylamin und |
| | ii) 2,4-Di-tert-octyl-4′-tert-butyldiphenylamin; |

insbesondere ein Gemisch enthaltend

a) 3 % Diphenylamin,

b) 14 %o 4-tert.Butyl-diphenylamin,

c) 30 % 4-tert.Octyl-diphenylamin, 4,4′-Di-tert.butyl-diphenylamin und 2,4,4′-Tri-tert.-butyl-diphenylamin,

d) 29 % 4-tert.Butyl-4′-tert.octyl-diphenylamin, 2,2′- und 3,3′-Di-tert.octyl-diphenylamin und 2,4-Di-tert.butyl-4′-tert.octyl-diphenylamin,

e) 18 % 4,4′-Di-tert.octyl-diphenylamin,

f) 6 % 2,4-Di-tert.octyl-4′-tert.butyl-diphenylamin.

Bevorzugte Verbindungen der Formel III sind die, worin $R_9$ und $R_{10}$ unabhängig voneinander Wasserstoff oder $C_1$-$C_{12}$-Alkyl bedeutet, und $R_{10'}$ Wasserstoff ist oder zusammen mit $R_{10}$ einen Butadiendiyl-Rest bildet, und $R_{11}$ und $R_{12}$ unabhängig voneinander $C_1$-$C_{12}$-Alkyl sind, oder $R_{11}$ und $R_{12}$ zusammen mit dem Kohlenstoffatom, an welches sie gebunden sind, einen $C_5$-$C_7$-Spiro-Cycloalkylring bilden, und $R_{13}$ Wasserstoff und $R_{14}$ $C_1$-$C_{12}$-Alkyl ist, oder $R_{13}$ und $R_{14}$ zusammen mit den beiden Kohlenstoffatomen, an die sie gebunden sind, einen Cyclohexanrest bilden, insbesondere jedoch die, worin $R_9$ Wasserstoff oder $C_1$-$C_{12}$-Alkyl ist, $R_{10}$ Wasserstoff, Methyl oder Ethyl ist, $R_{10'}$ Wasserstoff ist oder zusammen mit $R_{11}$ einen Butadiendiyl-Rest bildet und $R_{11}$ und $R_{12}$ Methyl oder Ethyl sind oder $R_{12}$ und $R_{13}$ zusammen mit dem Kohlenstoffatom, an welches sie gebunden sind, einen Spiro-Cyclohexylring bilden und $R_{13}$ Wasserstoff und $R_{14}$ Methyl oder Ethyl ist.

Ganz speziell bevorzugt als Verbindung der Formel III ist 2,2,4-Trimethyl- 1,2,3,4-tetrahydrochinolin.

Die Herstellung von Verbindungen der Formel III erfolgt nach an sich bekannten und z.B. in der US-A 4,692,258 beschriebenen Methoden.

Bevorzugte Alkalidithiophosphate entsprechen der Formel IV,

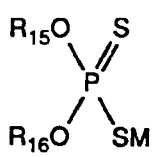

(IV)

worin $R_{15}$ und $R_{16}$ unabhängig voneinander $C_1$-$C_{24}$-Alkyl oder $C_2$-$C_{12}$-Alkyl, das durch -O-, -S- und/oder -C(O)O- unterbrochen ist; unsubstituiertes oder durch $C_1$-$C_{12}$-Alkyl substituiertes Phenyl; $C_5$-$C_{12}$-Cycloalkyl oder $C_5$-$C_{12}$-Cycloalkyl, das durch $C_1$-$C_4$-Alkyl substituiert ist; oder $C_7$-$C_{13}$-Phenylalkyl oder $C_7$-$C_{13}$-Phenylalkyl, das im Alkylrest mit -O- oder -S- unterbrochen ist, bedeutet, oder $R_{15}$ und $R_{16}$ zusammen eine Dimethylen-oder Trimethylengruppe oder eine Dimethylen- oder Trimethylengruppe, die durch $C_1$-$C_4$-Alkyl substituiert ist, bedeuten und worin M ein Alkalimetall darstellt; insbesondere von Interesse sind jedoch die, worin $R_{15}$ und $R_{16}$ unabhängig voneinander $C_3$-$C_8$-Alkyl oder durch $C_8$-$C_{12}$-Alkyl substituiertes Phenyl bedeuten und M Na oder K darstellt.

Stellen $R_{15}$ und $R_{16}$ $C_1$-$C_{24}$-Alkyl dar, so handelt es sich um geradkettige oder verzweigte Alkylreste, wie beispielsweise Methyl, Ethyl, n-Propyl, Isopropyl, n-Butyl, Isobutyl, sec.-Butyl, tert.-Butyl, 2-Methylpropyl, Pentyl, Hexyl, Heptyl, Octyl, 2-Ethylhexyl, Nonyl, Decyl, Undecyl, Dodecyl, Tetradecyl, Hexadecyl, Heptadecyl, Octadecyl oder Eicosyl. Bevorzugt sind Reste mit 3 bis 12 C-Atomen, besonders bevorzugt sind Reste mit 3 bis 8 C-Atomen.

Stellen $R_{15}$ und $R_{16}$ $C_2$-$C_{12}$-Alkyl dar, das durch -O-, -S- oder -C(O)O- unterbrochen ist, so kann das Heteroatom bzw. die C(O)O-Gruppe sich in jeder möglichen Position befinden, und der $C_2$-$C_{12}$-Alkylrest kann einfach oder mehrfach unterbrochen sein, wobei die Unterbrechung sowohl durch gleiche oder verschiedene Heteroatome als auch durch C(O)O-Gruppen erfolgen kann. Bevorzugt ist eine Unterbrechung.

Stellen $R_{15}$ und $R_{16}$ durch $C_1$-$C_{12}$-Alkyl substituiertes Phenyl dar, so kann der Phenylrest ein- oder mehrfach, bevorzugtjedoch ein- oder zweifach substituiert sein; bei $C_1$-$C_{12}$-Alkyl handelt es sich beispielsweise um Methyl, Ethyl, n-Propyl, Isopropyl, n-Butyl, Isobutyl, sec.-Butyl, tert.-Butyl, geradkettiges oder verzweigtes Nonyl oder Dodecyl. Bevorzugt ist einfach substituiertes Phenyl, wobei der Alkylrest zweckmässig 3- 12 C-Atome und bevorzugt 8- 12 C-Atome aufweist. Besonders zweckmässig ist Nonylphenyl.

Stellen $R_{15}$ und $R_{16}$ $C_5$-$C_{12}$-Cycloalkyl dar, so handelt es sich beispielsweise um Cyclopentyl, Cyclohexyl, Cycloheptyl, Cyclooctyl, Cyclononyl, Cyclodecyl, Cycloundecyl oder Cyclododecyl, vorzugsweise um Cyclohexyl.

Stellen $R_{15}$ und $R_{16}$ durch $C_1$-$C_4$-Alkyl substituiertes $C_5$-$C_{12}$-Cycloalkyl dar, so kann es sich um einfache oder mehrfache Substituenten, bevorzugt jedoch um einfache Substitution, handeln; wie beispielsweise um Methylcyclohexyl, Trimethylcyclohexyl, Butylcyclohexyl oder Propylcyclopentyl.

Stellen $R_{15}$ und $R_{16}$ $C_7$-$C_{13}$-Aralkyl dar, so handelt es sich beispielsweise um Benzyl, 1-oder 2-Phenylethyl, 3-Phenylpropyl, $\alpha,\alpha$-Dimethylbenzyl, 2-Phenylisopropyl, 2-Phenyl-hexyl, Benzhydryl oder Naphthylmethyl, vorzugsweise jedoch um Benzyl.

Stellen $R_{15}$ und $R_{16}$ $C_7$-$C_{13}$-Aralkyl dar, das im Alkylrest mit -O- oder -S- unterbrochen ist, so ist ein typisches Beispiel dafür eine Phenoxyethylgruppe.

Stellen $R_{15}$ und $R_{16}$ zusammen eine Dimethylen- oder Trimethylengruppe dar, die durch $C_1$-$C_4$-Alkyl substituiert ist, so trägt die Dimethylen- oder Trimethylengruppe zweckmässig eine, zwei oder drei Alkylgruppen mit 1, 2, 3 oder 4- C-Atomen und bevorzugt eine oder zwei Alkylgruppen mit 1, 2 oder 4 C-Atomen.

M stellt ein Alkalimetall dar, z.B. Li, Na, K oder Rb. Bevorzugte Metalle M sind Na und K, insbesondere Na.

Ganz speziell bevorzugt werden Verbindungen der Formel IV, worin $R_{15}$ und $R_{16}$ unabhängig voneinander 2-Methylpropyl oder 2-Ethylhexyl bedeuten.

Insbesondere bevorzugt handelt es sich bei den Verbindungen der Formel IV um O,O-Bis-2-methylpropylnatriumdithiophosphat oder O,O-Bis-2-ethylhexylnatriumdithiophosphat.

Die Verbindungen der Formeln II, III und/oder IV werden den erfindungsgemässen Zusammensetzungen zweckmässig in einer Menge von 0,01 bis 5 Gew.-%, vorzugsweise in einer Menge von 0,05 bis 3 Gew.-% und ganz besonders bevorzugt in einer Menge von 0,1 bis 2 Gew.-%, jeweils bezogen auf den Schmierstoff oder die Hydraulikflüssigkeit, beigemischt, mit der Massgabe, dass die Zugabe der Verbindungen der Formeln I, II, III und/oder IV ingesamt 5 Gew.-%, bezogen auf den Schmierstoff oder die Hydraulikflüssigkeit, nicht übersteigt.

Zweckmässig werden die Verbindungen der Formel I einerseits und die Verbindungen der Formeln II, III und/oder IV andererseits in ungefähr gleichen Gewichtsanteilen zugegeben.

Daher betrifft die vorliegende Erfindung ebenfalls ein Verfahren zur Stabilisierung von Schmierstoffen oder Hydraulikflüssigkeiten gegen oxidativen oder thermischen Abbau, dadurch gekennzeichnet, dass man dem Schmierstoff oder der Hydraulikflüssigkeit mindestens eine Verbindung der Formel I allein oder zusammen mit mindestens einem aromatischen Amin und/oder mindestens einem Alkalidithiophosphat zugibt.

Die erfindungsgemässen Schmierstoffe und Hydraulikflüssigkeiten können zusätzlich andere Additive enthalten, die zugegeben werden, um die Grundeigenschaften von Schmierstoffen und Hydraulikflüssigkeiten noch weiter zu verbessern; dazu gehören: weitere Antioxidantien, Metallpassivatoren, Rostinhibitoren, Viskositätsindex-Verbesserer, Stockpunktemiedriger, Dispergiermittel, Detergentien, weitere Hochdruck-Zusätze und Antiverschleiss-Additive.

Beispielsweise ist eine Reihe solcher Verbindungen der nachfolgenden Auflistung zu entnehmen.

Beispiele für phenolische Antioxidantien

1.Alkylierte Monophenole

2,6-Di-tert-butyl-4-methylphenol, 2,6-Di-tert-butylphenol, 2-tert-Butyl-4,6-dimethylphenol, 2,6-Di-tert-butyl-4-ethylphenol, 2,6-Di-tert-butyl-4-n-butylphenol, 2,6-Di-tert-butyl-4-iso-butylphenol, 2,6-Di-cyclopentyl-4-methylphenol, 2-($\alpha$-Methylcyclohexyl)-4,6-dimethylphenol, 2,6-Di-octadecyl-4-methylphenol, 2,4,6-Tri-cyclohexylphenol, 2,6-Di-tert-butyl-4-methoxymethylphenol, o-tert-Butylphenol.

2. Alkylierte Hydrochinone

2,6-Di-tert-butyl-4-methoxyphenol, 2,5-Di-tert-butyl-hydrochinon, 2,5-Di-tert-amyl-hydrochinon, 2,6-Diphenyl-4-octadecyloxyphenol.

3. Hydroxylierte Thiodiphenylether

2,2′-Thio-bis-(6-tert-butyl-4-methylphenol), 2,2′-Thio-bis-(4-octylphenol), 4,4′-Thio-bis-(6-tert-butyl-3-methylphenol), 4,4′-Thio-bis-(6-tert-butyl-2-methylphenol).

4. Alkyliden-Bisphenole

2,2′-Methylen-bis-(6-tert-butyl-4-methylphenol), 2,2′-Methylen-bis-(6-tert-butyl-4-ethylphenol), 2,2′-Methylen-bis-[4-methyl-6-(α-methylcyclohexyl)-phenol], 2,2′-Methylen-bis-(4-methyl-6-cyclohexylphenol), 2,2′-Methylen-bis-(6-nonyl-4-methylphenol), 2,2′-Methylen-bis-(4,6-di-tert-butylphenol), 2,2′-Ethyliden-bis-(4,6-di-tert-butylphenol), 2,2′-Ethyliden-bis-(6-tert-butyl-4- oder -5-iso-butylphenol), 2,2′-Methylen-bis-[6-(α-methylbenzyl)-4-nonylphenol], 2,2′-Methylen-bis-[6-(α,α-dimethylbenzyl)-4-nonylphenol], 4,4′-Methylen-bis-(2,6-di-tert-butylphenol), 4,4′-Methylen-bis-(6-tert-butyl-2-methylphenol), 1,1-Bis-(5-tert-butyl-4-hydroxy-2-methylphenyl)-butan, 2,6-Di-(3-tert-butyl-5-methyl-2-hydroxybenzyl)-4-methylphenol, 1,1,3-Tris-(5-tert-butyl-4-hydroxy-2-methylphenyl)-3-n-dodecylmercaptobutan, Ethylenglycol-bis-[3,3-bis-(3′-tert-butyl-4′-hydroxyphenyl)-butyrat], Bis-(3-tert-butyl-4-hydroxy-5-methylphenyl)-dicyclopentadien, Bis-[2-(3′-tert-butyl-2′-hydroxy-5′-methyl-benzyl)-6-tert-butyl-4-methyl-phenyl]-terephthalat.

5. Benzylverbindungen

1,3,5-Tri-(3,5-di-tert-butyl-4-hydroxybenzyl)-2,4,6-trimethylbenzol, Bis-(3,5-di-tert-butyl-4-hydroxybenzyl)-sulfid, 3,5-Di-tert-butyl-4-hydroxybenzyl-mercaptoessigsäure-isotridecylester, 3,5-Di-t ert-butyl-4-hydroxybenzyl-mercaptoessigsäure-iso-octylester, Bis-(4-tert-butyl-3-hydroxy-2,6-dimethylbenzyl)-dithiol-terephthalat, 1,3,5-Tris-(3,5-di-tert-butyl-4-hydroxybenzyl)-isocyanurat, 1,3,5-Tris-(4-tert-butyl-3-hydroxy-2,6-dimethylbenzyl)-isocyanurat, 3,5-Di-tert-butyl-4-hydroxybenzyl-phosphonsäure-di-octadecylester, 3,5-Di-tert-butyl-4-hydroxybenzyl-phosphonsäure-monoethylester, Calcium-salz.

6. Acylaminophenole

4-Hydroxy-laurinsäureanilid, 4-Hydroxy-stearinsäureanilid, 2,4-Bis-octylmercapto-6-(3,5-di-tert-butyl-4-hydroxyanilino)-s-triazin, N-(3,5-di-tert-butyl-4-hydroxyphenyl)-carbaminsäureoctylester.

7. Ester der β-(3,5-Di-tert-butyl-4-hydroxyphenyl)-propionsäure

mit ein- oder mehrwertigen Alkoholen, wie z.B. mit Methanol, Isooctanol, Diethylenglycol, Octadecanol, Triethylenglycol, 1,6-Hexandiol, Pentaerythrit, Neopentylglycol, Tris-hydroxyethyl-isocyanurat, Thiodiethylenglycol, Bis-hydroxyethyl-oxalsäurediamid.

8. Ester der β-(5-tert-butyl-4-hydroxy-3-methylphenyl)-propionsäure

mit ein- oder mehrwertigen Alkoholen, wie z.B. mit Methanol, Diethylenglycol, Octadecanol, Triethylenglycol, 1,6-Hexandiol, Pentaerythrit, Neopentylglycol, Tris-hydroxyethyl-isocyanurat, Thiodiethylenglykol, Di-hydroxyethyl-oxalsäurediamid.

9. Amide der β-(3,5-Di-tert-butyl-4-hydroxyphenyl)-propionsäure,

wie z.B. N,N′-Bis-(3,5-di-tert-butyl-4-hydroxyphenylpropionyl)-hexamethylendiamin, N,N′-Bis-(3,5-di-tert-butyl-4-hydroxyphenylpropionyl)-trimethylendiamin, N,N′-Bis-(3,5-di-tert-butyl-4-hydroxyphenylpropionyl)-hydrazin.

Beispiele für aminische Antioxidantien:

N,N′-Di-isopropyl-p-phenylendiamin, N,N′-Di-sec-butyl-p-phenylendiamin, N,N′-Bis-(1,4-dimethyl-pentyl)-p-phenylendiamin, N,N′-Bis(1-ethyl-3-methyl-pentyl)-p-phenylendiamin, N,N′-Bis (1-methyl-heptyl)-p-phenylendiamin, N,N′-Dicyclohexyl-p-phenylendiamin, N,N′-Diphenyl-p-phenylendiamin, N,N′-Di-(naphthyl-2)-p-phenylendiamin, N-Isopropyl-N′-phenyl-p-phenylendiamin, N-(1,3-Dimethyl-butyl)-N′-phenyl-p-phenylendiamin, N-(1-Methyl-heptyl)-N′-phenyl-p-phenylendiamin, N-Cyclohexyl-N′phenyl-p-phenylendiamin, 4-(p-Toluol-sulfonamido)-diphenylamin, N,N′-Dimethyl-N,N′-di-sec-butyl-p-phenylendiamin, 4-n-Butylaminophenol, 4-Butyrylamino-phenol, 4-Nonanoylamino-phenol, 4-Dodecanoylamino-phenol, 4-Octadecanoylamino-phenol, 2,6-Di-tert-butyl-4-dimethylamino-methyl-phenol, 2,4′-Diamino-diphenylmethan, 4,4′-Diamino-diphenylmethan, N,N,N′,N′-Tetramethyl-4,4′-diamino-diphenylmethan, 1,2-Di-[(2-methyl-phenyl)-amino]-ethan, 1,2-Di-(phenylamino)-propan, (o-Tolyl)-biguanid, Di-[4-(1′,3′-dimethyl-butyl)-phenyl]amin, 2,3-Dihydro-3,3-dimethyl-4H-1,4-benzothiazin, Phenothiazin, N-Allylphenothiazin, N,N-Bis-(2,2,6,6-tetramethylpiperidin-4-yl-hexamethylendiamin, Bis-(2,2,6,6-tetramethylpiperidin-4-yl)-sebacat, 2,2,6,6-Tetramethylpiperidin-4-on, 2,2,6,6-Tetramethylpiperidin-4-ol.

Beispiele für weitere Antioxidantien:

Aliphatische oder aromatische Phosphite, Ester der Thiodipropionsäure oder der Thiodiessigsäure, oder Salze der Dithiocarbamid- oder Dithiophosphorsäure, 2,2,12,12-Tetramethyl-5,9-dihydroxy-3,7,11-trithiatridecan und 2,2,15,15-Tetramethyl-5,12-dihydroxy-3,7,10,14-tetrathiahexadecan.

Beispiele für Metall-Desaktivatoren, z.B. für Kupfer, sind:

Triazole, Benztriazole und deren Derivate, Tolutriazole und deren Derivate, 2-Mercaptobenzthiazol, 2-Mercaptobenztriazol, 2,5-Dimercaptobenztriazol, 2,5-Dimercaptobenzthiadiazol, 5,5'-Methylenbisbenztriazol, 4,5,6,7-Tetrahydrobenztriazol, Salicyliden-propylendiamin, Salicylaminoguanidin und dessen Salze, 1-[N,N-Bis(2-ethylhexyl)-amino-methyl]-5(6)-methyl-1H-benztriazol-1-(1-Cyclohexyloxy butyl-5(6)-methyl- 1H-triazol, 1-[N,N-Bis(2-ethylhexyl)aminomethyl]-1H-1,2,4-triazol.

Beispiele für Rost-Inhibitoren sind:

a) Organische Säuren, ihre Ester, Metallsalze und Anhydride, z.B.:
N-Oleoyl-sarcosin, Sorbitan-mono-oleat, Blei-naphthenat, Alkenylbemsteinsäure-anhydrid, z.B. Dodecenylbemsteinsäure-anhydrid, Alkenylbemsteinsäure-Teilester und -Teilamide, 4-Nonylphenoxyessigsäure, 2-(2-Carboxyethyl)-1-dodecyl-3-methylglycerin und dessen Salze, insbesondere Na- und Triethanolaminsalze.
b) Stickstoffhaltige Verbindungen, z.B.:
I.Primäre, sekundäre oder tertiäre aliphatische oder cycloaliphatische Amine und Amin-Salze von organischen und anorganischen Säuren, z.B. öllösliche Alkylammoniumcarboxylate, ferner 1[N,N-bis-(2-hydroxyethyl)amino]-3-(4-nonyl-phenoxy)propan-2-ol.
II. Heterocyclische Verbindungen, z.B.:
Substituierte Imidazoline und Oxazoline.
c) Phosphorhaltige Verbindungen, z.B.:
Aminsalze von Phosphorsäurepartialestem oder Phosphonsäurepartialestem, Zinkdi-alkyldithiophosphate.
d) Schwefelhaltige Verbindungen, z.B.:
Barium-dinonylnaphthalin-sulfonate, Calciumpetroleum-sulfonate.

Beispiele für Viskositätsindex-Verbesserer sind:

Polyacrylate, Polymethacrylate, Vinylpyrrolidon/Methacrylat-Copolymere, Polyvinylpyrrolidone, Polybutene, Olefin-Copolymere, Styrol/Acrylat-Copolymere, Polyether.

Beispiele für Stockpunktemiedriger sind:

Polymethacrylat, alkylierte Naphthalinderivate.

Beispiele für Dispergiermittel/Tenside sind:

Polybutenylbemsteinsäureamide oder -imide, Polybutenylphosphonsäurederivate, basische Magnesium-, Calcium-, und Bariumsulfonate und -phenolate.

Beispiele für Verschleissschutz-Additive sind:

Schwefel und/oder Phosphor und/oder Halogen enthaltende Verbindungen, wie geschwefelte pflanzliche Oele, Zinkdialkyldithiophosphate, Tritolylphosphat, chlorierte Paraffine, Alkyl- und Aryldi- und tri-sulfide, Triphenylphosphorothionate, Diethanol-aminomethyltolyltriazol, Di(2-ethylhexyl)aminomethyltolyltriazol, 3-[(Bis-isopropyl-oxy-phosphinothioyl)thio] -propionsäure-ethylester, Gemische von Alkylphenyl-phosphorothioaten, Triphenylthiophosphat (Triphenylphosphorothioat), Dodecylaminsalz des 3-Hydroxy- 1,3-thiaphosphetan-3-oxids, Trithiophosphorsäure-5,5,5-tris[isooctylacetat (2)], 1-[N,N-Bis(2-ethylhexyl)aminomethyl-2-mercapto-1H-1,3-benzthiazol.

Die Verbindungen der Formel I sind zum Teil bekannt, wie bereits weiter oben ausgeführt wurde. Neu sind hingegen Verbindungen der Formel I, worin $R_1$ $-NR_5R_6$ bedeutet.

Gegenstand der vorliegenden Erfindung sind daher auch Verbindungen der Formel I, worin X 1, und n 2 und R bzw. $R_1$ $-NR_5R_6$ bedeuten, wobei $R_5$ und $R_6$ sowie $R_2$ und $R_3$ wie oben unter Formel I definiert sind.

In bevorzugten neuen Verbindungen der Formel I sind $R_2$ und $R_3$ einerseits und $R_5$ und $R_6$ andererseits gleich. Zweckmässig sind auch solche Verbindungen, in denen $-NR_2R_3$ und $-NR_5R_6$ gleich sind.

In zweckmässigen neuen Verbindungen der Formel I bedeuten $R_2$, $R_3$, $R_5$ und $R_5$, die gleich oder verschieden sein können, $C_1$-$C_{12}$-Alkyl.

Anhand der nachfolgenden Beispiele wird die Erfindung weiter erläutert. Alle Angaben in Prozenten oder Teilen beziehen sich auf das Gewicht, sofern nicht anders angegeben.

Beispiel 1: Zu einer Lösung von 9,74 g (0,04 Mol) 1,1-Diethyl-3-benzoyl-thioharnstoff in 600 ml Methanol wird bei 50°C eine gesättigte Lösung von 4,0 g (0,02 Mol) Kupfer(II)acetat Monohydrat in 150 ml Methanol zugetropft. Das dunkelgrün-schwarze Reaktionsgemisch wird 3 h bei 50-60°C gerührt und auf ca. 1/3 seines Volumens aufkonzentriert. Beim Abkühlen auf 4°C bilden sich glänzende olive-schwarze Kristalle der Formel

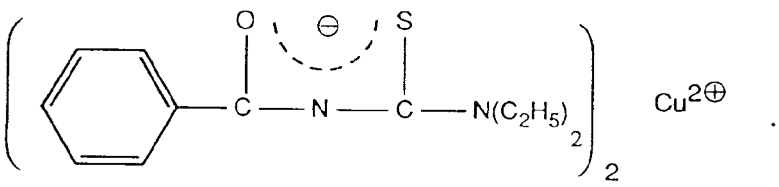

Ausbeute 9,5 g (88,8 % d. Th.) mit einem Smp. von 102°C.

Analyse $C_{24}H_{30}N_4O_2S_2Cu$:

| | C | H | N | S | Cu |
|---|---|---|---|---|---|
| ber.% | 53,96 | 5,66 | 10,49 | 12,00 | 11,90 |
| gef.% | 53,98 | 5,70 | 10,49 | 11,91 | 12,0 |

Beispiele 2-7: Analog zu der in Beispiel 1 beschriebenen Methode werden die aus der Tabelle 1 ersichtlichen Verbindungen der Formel I hergestellt.

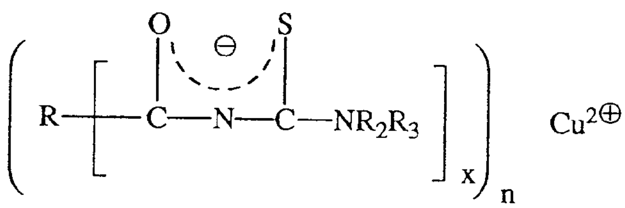

Tabelle 1:

| Bsp. | x | n | R | $NR_2R_3$ | Smp. [°C] | Ausbeute [% d. Th.] | Aspekte | Analyse [berechnet / gefunden %] C | H | N | S | Cu |
|---|---|---|---|---|---|---|---|---|---|---|---|---|
| 2 | 1 | 2 | 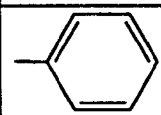 | $-N(C_3H_7)_2$ | 107-108 | 99 | grünschwarze Kristalle | 56,21<br>56,78 | 7,07<br>6,52 | 9,37<br>9,25 | 10,72<br>11,02 | 10,62<br>10,80 |
| 3 | 1 | 2 | 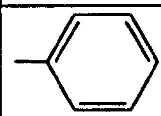 | $-N(C_4H_9)_2$ | 95-97 | 92 | schwarzes Pulver | 59,46<br>59,36 | 7,17<br>7,17 | 8,67<br>8,61 | 9,92<br>10,05 | 9,83<br>9,90 |
| 4 | 1 | 2 | 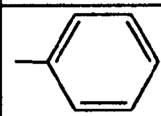 | $-N(i-C_4H_9)_2$ | 158-159 | 94 | schwarzes Pulver | 59,46<br>59,46 | 7,17<br>7,14 | 8,67<br>8,63 | 9,92<br>9,92 | 9,83<br>10,0 |
| 5 | 1 | 2 | 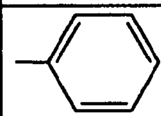 | $-N(C_6H_{13})_2$ | - | 99 | viskoses schwarzes Oel | 63,33<br>64,80 | 8,24<br>8,16 | 7,39<br>7,08 | 8,45<br>8,00 | 8,38<br>7,65 |
| 6 | 1 | 2 | 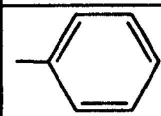 | $-N(2\text{-Ethylhexyl})_2$ | - | 46 | viskoses dunkelbraunes Oel | 66,20<br>68,75 | 9,03<br>9,21 | 6,43<br>5,84 | 7,36<br>6,98 | 7,30<br>6,18 |
| 7 | 2 | 1 | $-(CH_2)_4-$ | $-N(2\text{-Ethylhexyl})_2$ | - | 75 | grünschwarzes hochviskoses Oel | 62,17<br>62,63 | 9,91<br>9,75 | 7,25<br>6,84 | 8,30<br>8,38 | 8,22<br>7,88 |

Beispiel 8: Zu einer Lösung von 119 g (0,29 Mol) Tetrabutylthiuramdisulfid (MERAMID TBTD) in 400 ml Toluol werden bei 40°C innerhalb 50 min. 18,9 ml (31,5 g, 0,23 Mol) Sulfurylchlorid zugetropft. Die Suspension wird 4 Std. bei 60°C gerührt, abgekühlt, filtriert und eingedampft. Das heterogene Rohprodukt wird zur Reinigung in 400 ml Hexan aufgenommen, filtriert und eingedampft. Man erhält 120 g eines hellbraunen Oels, das bei 105- 112°C und 0,01 Torr destilliert wird. Man erhält 66 g Dibutylthiocarbamylchlorid (55 % d. Th.) als

hellbraunes Oel der Formel

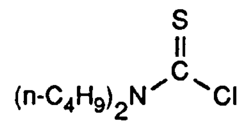

Zu einer Lösung von 20,8 g (0,1 Mol) Dibutylthiocarbamylchlorid in 90 ml Aceton werden 9,7 g (0,1 Mol) Kaliumthiocyanant zugegeben. Die Suspension wird 15 min. am Rückfluss gerührt, abgekühlt und filtriert. Zu der filtrierten Lösung werden bei 15-20°C innerhalb 15 min. 17 ml (12,9 g, 0,1 Mol) Dibutylamin zugetropft. Nach 1 Std. Rühren bei Raumtemperatur werden bei 0-5°C innerhalb 15 min. 11,4 ml Bromwasserstoff 48 % (16,9 g, 0,1 Mol) und innerhalb 30 min. 11,3 g Wasserstoffperoxid 30 % (0,1 Mol) zugetropft. Dann wird 1 Std. bei 10- 15°C und 1 Std. bei Raumtemperatur gerührt. Zu der orange trüben Lösung werden schliesslich bei 20-30°C innerhalb 30 min. 50 ml Natronlauge 2N (0,1 Mol) zugetropft. Bei Raumtemperawt wird 1 Std. nachgerührt. Die organische Phase wird abgetrennt, dreimal mit je 100 ml Wasser gewaschen (pH 7), über Natriumsulfat getrocknet, filtriert und eingedampft: 32 g dunkelbraunes Oel. Dieses Rohprodukt wird mit Dichlormethan über 200 g Silicagel chromatographisch gereinigt. Man erhält so 10 g 1,1,5,5-Tetrabutyl-2-thiobiuret als dunkeloranges, mittelviskoses Oel der Formel

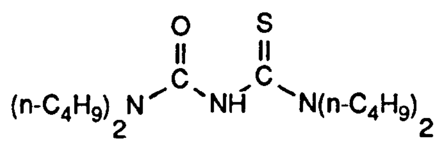

Zu einer Lösung von 1,7 g (5 mMol) 1,1,5,5-Tetrabutyl-2-thiobiuret in 20 ml Toluo/Ethanol (1:1) wird bei 50°C innerhalb 10 min. eine Lösung von 0,65 g (2,5 mMol) Kupfer(II)-acetylacetonat in 10 ml Toluo/Ethanol (1:1) zugetropft. Nach 1 Std. bei 50°C wird filtriert und eingedampft. 2,3 g Rohprodukt als hochviskoses, dunkles Oel werden mit Dichlormethan über 16 g Silicagel chromatographisch gereinigt. Man erhält so 1,0 g Kupferkomplex als dunkelgrünes, mittelviskoses Oel (53 % d. Th.) der Formel

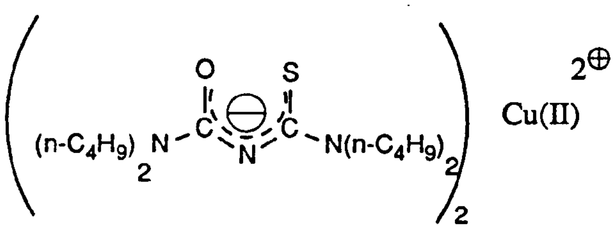

Beispiel 9: Verfährt man wie in Beispiel 8, geht jedoch von Tetra(2-ethylhexyl)thiuramdisulfid aus und verwendet an Stelle von Dibutylamin Di(2-ethylhexyl)amin, so erhält man die Verbindung der Formel

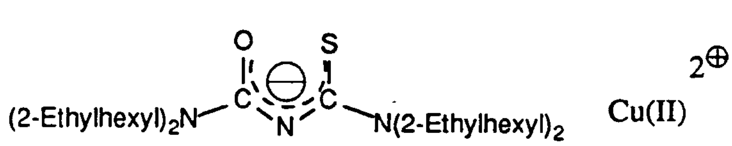

Analysendaten:

| Verbindung von Bsp. Nr. | Aspekt | Analyse [berechnet gefunden] C | H | N | S | Cu |
|---|---|---|---|---|---|---|
| 8 | dunkelgrünes mittelviskoses Oel | 57.60<br>58.17 | 9.94<br>9.55 | 11.19<br>10.97 | 8.54<br>9.30 | 8.46<br>8.18 |
| 9 | dunkelgrünes hochviskoses Oel | 68.20<br>69.03 | 11.45<br>11.22 | 7.02<br>6.74 | 5.36<br>5.63 | 5.31<br>5.07 |

Beispiele 10-16: Thermische Stabilisierung eines synthetischen Oeles. Die thermische Alterung der Formulierungen wird in einem Druck-Differenz-Kalorimeter (Pressure-Differential-Scanning Calorimetry, PDSC) durchgeführt.

Das Verfahren arbeitet nach dem folgenden Prinzip: Die PDSC-Zelle (Thermoanalysen-system 1090 von DuPont) besteht aus einem Heizblock aus Silber. In diesen Heizblock ist eine Konstantanplatte eingesetzt, welche die Thermoelemente (Chromel-Alumel) enthält. Auf die etwas erhöht angebrachten Thermoelemente werden Probepfännchen und Referenzpfännchen aus Aluminium gestellt. Der Innenraum der DSC-Zelle ist mit einem dünnen Goldfilm überzogen (Korrosionsschutz). Das Referenzpfännchen bleibt leer, in das Probepfännchen werden drei Tropfen der jeweiligen Formulierung eingefüllt. Bestimmt wird die Temperaturdifferenz zwischen Proben- und Referenzpfännchen unter isothermen Bedingungen. Die Enthalpieänderung dH/dt wird jeweils in mW angegeben. Alle Messungen werden in Sauerstoff durchgeführt. Die Temperatur beträgt 180°C isotherm. Der Druck beträgt 10 bar. Als Basisöl wird jeweils ein kommerziell erhältliches Mineralöl (Mobil 150 SSU) eingesetzt. Um die Oxidationsanfälligkeit des Oeles zu verstärken, werden diesem Oel 40 ppm $Fe^{3\oplus}$ zugefügt. Zur Prüfung der Wirksamkeit enthält das Oel die in Tabelle 2 angeführten Additiven in der angegebenen Menge.

Während der thermischen Alterung nimmt die Konzentration der zugefügten Additive laufend ab. Bei einer kritischen Additivkonzentration steigt die Wärmetönung dH/dt an. Die Zeit, die verstreicht bis dieser Anstieg erfolgt, wird als Induktionszeit (onset) bezeichnet. Demnach deuten lange Induktionszeiten auf eine hohe Alterungsstabilität der Oele hin.

Es werden die folgenden aus der Tabelle 2 ersichtlichen Formulierungen hergestellt und gemessen.

Tabelle 2:

| Bsp. | Additiv Verbindung von Bsp. | Konz. [%] | Oel | Induktionszeit [min] |
|---|---|---|---|---|
| 10 | 3 | 0,5 | Basisöl | 25,6 |
| 11 | 6 | 0,5 | Basisöl | 16,2 |
| 12 | 3 + Amin-AO* | 0,2 0,3 | Basisöl | 37,5 |
| 13 | 3 + Amin-AO* | 0,1 0,4 | Basisöl | 50,5 |
| 14 | 6 + Amin-AO* | 0,2 0,3 | Basisöl | 31,7 |
| 15 | 8 + Amin-AO* | 0,1 0,4 | Basisöl | 57 |
| 16 | 9 + Amin-AO* | 0,1 0,4 | Basisöl | 33 |
| - | — | | Basisöl | < 1 |

* Amin-AO: Technisches Gemisch erhalten durch Reaktion von Diphenylamin mit Diisobutylen, entsprechend der Zusammensetzung

a) 3 % Diphenylamin,

b) 14 % 4-tert.Butyl-diphenylamin,

c) 30 % 4-tert.Octyl-diphenylamin, 4,4'-Di-tert.butyl-diphenylamin und 2,4,4'-Tri-tert.butyl-diphenylamin,

d) 29 % 4-tert.Butyl-4'-tert.octyl-diphenylamin, 2,2'- und 3,3'-Di-tert.octyl-diphenylamin und 2,4-Di-tert.butyl-4'-tert.octyl-diphenylamin,

e) 18 % 4,4'-Di-tert.octyl-diphenylamin,

f) 6 % 2,4-Di-tert.octyl-4'-tert.butyl-diphenylamin.

Beispiel 17: Mit der gleichen PDSC-Methode wie in den Beispielen 10- 16 wird für eine Formulierung enthaltend eine Kombination der Verbindung gemäss Beispiel 3 und O,O-Bis-2-ethylhexyl-natriumdithiophosphat (Na-DTP) die Induktionszeit bestimmt. Die Resultate sind in Tabelle 3 wiedergegeben.

Tabelle 3:

| Additiv Verbindung von Bsp. | Konz. [%] | Oel | Induktionszeit [min] |
|---|---|---|---|
| 3 + Na-DTP | 0,25 0,25 | Basisöl | 23,6 |
| 3 | 0,5 | Basisöl | 25,6 |
| Na-DTP | 0,5 | Basisöl | 5,7 |
| — | | Basisöl | < 1 |

Beispiel 18: Die Antioxidanswirkung wird mit einem kommerziellen Schwing-Reib-Verschleissgerät (SRV-Gerät) der Firma Optimol GmbH, München bestimmt.

Das Verfahren ist ausführlich beschrieben in R. Schumacher, D. Landolt, H.J. Mathieu und H. Zinke, Surface Reaction of Isogeometrical Phosphorus Compounds, ASLE Transaction, 26 (1982) 94-101.

Dieses Gerät basiert auf dem folgenden Prinzip: Eine Stahlkugel ( 100 Cr 6), auf die eine Kraft $F_N$ wirkt, oszilliert auf einem Stahlzylinder. Die Kugel ist in einer Halterung fixiert und führt demnach eine oszillierende Gleitbewegung aus. Die Horizontal- und Vertikalkraft wird durch einen piezoelektrischen Kraftaufnehmer bestimmt. Unter den vorliegenden Versuchsbedingungen beträgt die maximale Hertz'sche Normalspannung 2740 N/mm$^2$, die maximale Schubspannung 850 N/mm$^2$. Kugel und Zylinder sind aus demselben Werkzeugstahl hergestellt worden.

Einige Tropfen Oel, welches das zu untersuchende Gemisch gelöst enthält, werden zwischen Zylinder und Kugel aufgebracht. Die folgenden Testbedingungen werden gewählt:

Prüfbedingungen:

| | |
|---|---|
| Last | 50 N |
| Temperatur | 200°C |
| Frequenz | 50 Hz |
| Amplitude | 1000 μm |
| Prüfdauer | Bis zur Abschaltung des Geräts wegen Ueberbeanspruchung (Oelverdickung wegen oxidativem Abbau). Lange Prüfdauer bedeutet gute Antioxidans-Wirkung. Die Resultate sind in Tabelle 4 zusammengestellt. |

Als Basisöl dient ein "low reference"-Oel für Dieselmotoren

Tabelle 4

| Zusammensetzung | Prüfdauer [h] |
|---|---|
| Basisöl + 0,2 % 3* + 0,3 % Na-DTP** | 42 |
| Basisöl | 35 |

*Verbindung gemäss Beispiel 3

**Siehe Beispiel 17

Beispiel 19: Analog Beispiel 18 wird im gleichen Basisöl die Formulierung aus Beispiel 12 geprüft. Die Prüfdauer bis zum Abschalten des Geräts wegen Ueberbelastung infolge Oelverdickung beträgt 45 h.

## Patentansprüche

1. Zusammensetzung enthaltend
   a) einen Schmierstoff oder eine Hydraulikflüssigkeit und
   b) mindestens eine Verbindung der allgemeinen Formel I

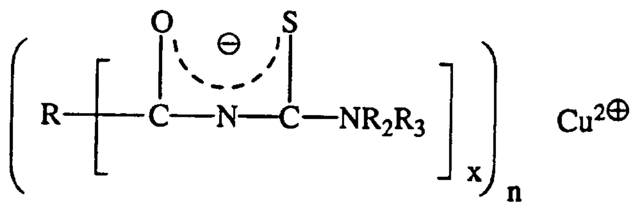

(I),

wobei x=1 oder 2 ist und, wenn x=1 ist, n=2 ist und R die Bedeutung von $R_1$ hat, oder, wenn x=2 ist, n=1 ist und R die Bedeutung von $R_4$ hat, und $R_1$ Alkyl mit 1-25 C-Atomen, eine unsubstituierte oder $C_1$-$C_8$-alkylsubstituierte Cycloalkylgruppe mit 5-12 C-Atomen, Alkenyl mit 2-18 C-Atomen, Phenyl, Naphthyl, $C_7$-$C_{18}$-Phenylalkyl, $C_7$-$C_{18}$-Alkylphenyl oder $-NR_5R_5$ bedeutet, und $R_2$ und $R_3$ gleich oder verschieden sind und Alkyl mit 1-18 C-Atomen, Phenyl, Naphthyl, $C_7$-$C_{18}$-Phenylalkyl oder $C_7$-$C_{18}$-Alkylphenyl bedeuten, oder $R_2$ und $R_3$, zusammen mit dem sie verbindenden N-Atom, einen 5- bis 7-gliedrigen heterozyklischen Ring bilden, der gegebenenfalls zusätzlich ein N- oder O-Atom enthalten kann, $R_4$ Alkylen mit 1-12 C-Atomen, das gegebenenfalls durch mindestens eine -O-Gruppe unterbrochen ist, darstellt oder Alkenylen mit 2-12 C-Atomen ist, das gegebenenfalls durch mindestens eine -O-Gruppe unterbrochen ist, und $R_5$ und $R_5$ die gleichen Bedeutungsmöglichkeiten wie $R_2$ und $R_3$ haben.

2. Zusammensetzung gemäss Anspruch 1, worin $R_1$ Alkyl mit 1-18 C-Atomen, eine unsubstituierte Cycloalkylgruppe mit 5-8 C-Atomen, Phenyl, $C_7$-$C_{12}$-Alkylphenyl, $C_7$-$C_{12}$-Phenylalkyl oder $-NR_5R_6$ bedeutet, $R_2$ und $R_3$ bzw. $R_5$ und $R_6$ gleich oder verschieden sind und jeweils Alkyl mit 1-18 C-Atomen oder Phenyl bedeuten, oder $R_2$ und $R_3$ bzw. $R_5$ und $R_5$ zusammen mit dem sie verbindenden N-Atom eine Piperidino-, Hexamethylenimino-, Piperazino- oder Morpholinogruppe bilden, und $R_4$ Alkylen mit 1-12 C-Atomen oder Alkenylen mit 2- 12 C-Atomen darstellt.

3. Zusammensetzung gemäss Anspruch 1, worin $R_1$ Alkyl mit 1-18 C-Atomen, Phenyl, $-NR_5R_5$ oder Benzyl darstellt, und $R_2$ und $R_3$ bzw. $R_5$ und $R_5$ gleich oder verschieden sind und jeweils Alkyl mit 1-18 C-Atomen bedeuten.

4. Zusammensetzung gemäss Anspruch 1, worin $R_1$ Alkyl mit 6-18 C-Atomen, $-NR_5R_6$ oder Phenyl darstellt, $R_2$ und $R_3$ bzw. $R_5$ und $R_5$ unabhängig voneinanderjeweils Alkyl mit 1-12 C-Atomen und $R_4$ Alkylen mit 2-10 C-Atomen bedeuten.

5. Zusammensetzung gemäss Anspruch 1, worin $R_1$ Phenyl oder $-NR_5R_6$ darstellt, und $R_2$ und $R_3$ bzw. $R_5$ und $R_5$ unabhängig voneinanderjeweils Alkyl mit 1-8 C-Atomen bedeuten.

6. Zusammensetzung gemäss Anspruch 1, worin $R_2$ und $R_3$ unabhängig voneinander Alkyl mit 4-8 C-Atomen darstellen, und $R_4$ Alkylen mit 1-10 C-Atomen oder Alkenylen mit 2-10 C-Atomen bedeutet.

7. Zusammensetzung gemäss Anspruch 1, worin $R_2$ und $R_3$ unabhängig voneinander Alkyl mit 4-8 C-Atomen darstellen, und $R_4$ Alkylen mit 3-8 C-Atomen bedeutet.

8. Zusammensetzung gemäss Anspruch 2, worin, wenn x=1 ist, $R_1$ Phenyl oder $-NR_5R_5$ bedeutet, und $R_2$ und $R_3$ bzw. $R_5$ und $R_5$ gleich sind und je Alkyl mit 1-12 C-Atomen darstellen, oder worin, wenn x=2 ist, $R_4$ Alkylen mit 3-8 C-Atomen ist und $R_2$ und $R_3$ je Alkyl mit 3- 10 C-Atomen bedeuten.

9. Zusammensetzung gemäss Anspruch 1, die c) zusätzlich mindestens ein aromatisches Amin oder/und

mindestens ein Alkalidithiophosphat enthält.

**10.** Zusammensetzung gemäss Anspruch 9, worin das aromatische Amin eine Verbindung der Formeln II oder III oder eine Mischung von solchen Verbindungen ist,

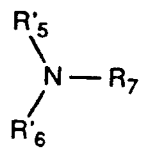 (II) 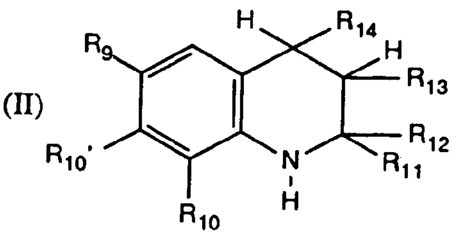 (III)

worin $R'_5$ $C_1$-$C_{18}$-Alkyl, $C_7$-$C_9$-Phenylalkyl, $C_5$-$C_{12}$-Cycloalkyl, Phenyl, $C_7$-$C_{18}$-Alkylphenyl, $C_7$-$C_{18}$-Alkoxyphenyl oder Naphthyl bedeutet,
$R'_6$ Phenyl, $C_7$-$C_{18}$-Alkylphenyl, $C_7$-$C_{18}$-Alkoxyphenyl oder Naphthyl bedeutet,
$R_7$ Wasserstoff, $C_1$-$C_{12}$-Alkyl, Benzyl, Allyl, Methallyl, Phenyl oder eine Gruppe $-CH_2SR_8$ bedeutet, und
$R_8$ $C_4$-$C_{18}$-Alkyl, $-CH_2COO(C_4$-$C_{18}$-Alkyl) oder $-CH_2CH_2COO(C_4$-$C_{18}$-Alkyl) bedeutet, und
$R_9$ und $R_{10}$ unabhängig voneinander Wasserstoff, $C_1$-$C_{18}$-Alkyl oder Benzyl bedeuten, und
$R_{10'}$ Wasserstoff oder $C_1$-$C_{12}$-Alkyl bedeutet oder zusammen mit $R_{10}$ einen Butadiendiyl-Rest bildet, und
$R_{11}$ und $R_{12}$ unabhängig voneinander $C_1$-$C_{18}$-Alkyl, Phenyl oder Benzyl sind oder $R_{11}$ und $R_{12}$ zusammen mit dem Kohlenstoffatom, an welches sie gebunden sind, einen $C_5$-$C_{12}$-Spiro-Cycloalkylring bilden, und
$R_{13}$ Wasserstoff oder $C_1$-$C_{18}$-Alkyl und
$R_{14}$ $C_1$-$C_{18}$-Alkyl ist, oder
$R_{13}$ und $R_{14}$ zusammen mit den beiden Kohlenstoffatomen, an die sie gebunden sind,
einen $C_5$-$C_{12}$-cycloaliphatischen Rest bedeuten enthalten sind.

**11.** Zusammensetzung gemäss Anspruch 10, worin die Verbindung der Formel II ein technisches Gemisch ist, erhalten durch Reaktion von Diphenylamin mit Diisobutylen, enthaltend

| | |
|---|---|
| 1 bis 5 Gew.-% | a) Diphenylamin |
| 8 bis 18 Gew.-% | b) 4-tert-Butyldiphenylamin |
| 21 bis 31 Gew.-% | c) einer oder mehrerer der Verbindungen |
| | i) 4-tert-Octyldiphenylamin |
| | ii) 4,4′-Di-tert-butyldiphenylamin |
| | iii) 2,4,4′-Tris-tert-butyldiphenylamin, |
| 20 bis 31 Gew.-% | d) einer oder mehrerer der Verbindungen |
| | i) 4-tert-Butyl-4′-tert-octyldiphenylamin |
| | ii) 2,2′- oder 2,4′-Di-tert-octyldiphenylamin |
| | iii) 2,4-Di-tert-butyl-4′-tert-octyldiphenylamin und |
| 15 bis 29 Gew.-% | e) der Verbindung |
| | i) 4,4′-Di-tert-octyldiphenylamin oder der Verbindungen |
| | i) 4,4′-Di-tert-octyldiphenylamin und |
| | ii) 2,4-Di-tert-octyl-4′-tert-butyldiphenylamin. |

**12.** Zusammensetzung gemäss Anspruch 9, worin das Alkalidithiophosphat eine Verbindung der Formel IV ist,

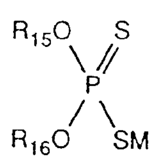 (IV)

worin $R_{15}$ und $R_{16}$ unabhängig voneinander $C_1$-$C_{24}$-Alkyl oder $C_2$-$C_{12}$-Alkyl, das durch -O-, -S- und/oder -C(O)O- unterbrochen ist; unsubstituiertes oder durch $C_1$-$C_{12}$-Alkyl substituiertes Phenyl; $C_5$-$C_{12}$-Cyclo-

alkyl oder $C_5$-$C_{12}$-Cycloalkyl, das durch $C_1$-$C_4$-Alkyl substituiert ist; oder $C_7$-$C_{13}$-Phenylalkyl oder $C_7$-$C_{13}$-Phenylalkyl, das im Alkylrest mit -O- oder -S- unterbrochen ist, bedeutet, oder $R_{15}$ und $R_{16}$ zusammen eine Dimethylen- oder Trimethylengruppe oder eine Dimethylen- oder Trimethylengruppe, die durch $C_1$-$C_4$-Alkyl substituiert ist, bedeuten und worin M ein Alkalimetall darstellt.

**13.** Verfahren zur Stabilisierung von Schmierstoffen oder Hydraulikflüssigkeiten gegen oxidativen oder/und thermischen Abbau, dadurch gekennzeichnet, dass man dem Schmierstoff oder der Hydraulikflüssigkeit mindestens eine Verbindung der Formel I nach Anspruch 1 zugibt.

**14.** Verbindungen der in Anspruch 1 definierten Formel I, worin x = 1, n = 2 und R bzw. $R_1$ eine Gruppe $-NR_5R_6$ bedeuten, wobei $R_5$ und $R_6$ wie in Anspruch 1 definiert sind und $R_2$ und $R_3$ die in Anspruch 1 angegebene Bedeutung haben.

**15.** Verbindungen nach Anspruch 14, worin $R_2$ und $R_3$ sowie $R_6$ und $R_6$ jeweils unabhängig voneinander $C_1$-$C_{12}$-Alkyl bedeuten.

## Claims

**1.** A composition comprising
a) a lubricant or a hydraulic fluid, and
b) at least one compound of general formula I

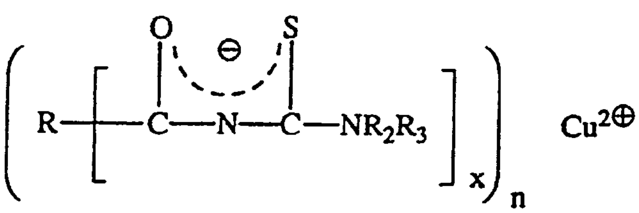

(I),

wherein x is 1 or 2 and, when x is 1, n is 2 and R has the meaning of $R_1$, or, when x is 2, n is 1 and R has the meaning of $R_4$, and $R_1$ is alkyl of 1 to 25 carbon atoms, an unsubstituted or $C_1$-$C_8$alkyl-substituted cycloalkyl group of 5 to 12 carbon atoms, alkenyl of 2-18 carbon atoms, phenyl, naphthyl, $C_7$-$C_{18}$phenylalkyl, $C_7$-$C_{18}$alkylphenyl or $-NR_5R_6$, and $R_2$ and $R_3$ are identical or different and are alkyl of 1 to 18 carbon atoms, phenyl, naphthyl, $C_7$-$C_{18}$phenylalkyl or $C_7$-$C_{18}$alkylphenyl, or $R_2$ and $R_3$, together with the linking N atom, form a 5- to 7-membered heterocyclic ring which may additionally contain an N or O atom, $R_4$ is alkylene of 1 to 12 carbon atoms which may be interrupted by at least one -O- group, or is alkenylene of 2 to 12 carbon atoms which may be interrupted by at least one -O-group, and $R_6$ and $R_6$ have the same possible meanings as $R_2$ and $R_3$.

**2.** A composition according to claim 1, wherein $R_1$ is alkyl of 1 to 18 carbon atoms, an unsubstituted cycloalkyl group of 5 to 8 carbon atoms, phenyl, $C_7$-$C_{12}$alkylphenyl, $C_7$-$C_{12}$phenylalkyl or $-NR_5R_6$, $R_2$ and $R_3$ and $R_6$ and $R_6$ are identical or different and are each alkyl of 1 to 18 carbon atoms or phenyl, or $R_2$ and $R_3$ and $R_5$ and $R_6$ respectively, together with the linking N atom, form a piperidino, hexamethyleneimino, piperazino or morpholino group, and $R_4$ is alkylene of 1 to 12 carbon atoms or alkenylene of 2 to 12 carbon atoms.

**3.** A composition according to claim 1, wherein $R_1$ is alkyl of 1 to 18 carbon atoms, phenyl, $-NR_5R_6$ or benzyl, and $R_2$ and $R_3$ and $R_6$ and $R_6$ are identical or different and are each alkyl of 1 to 18 carbon atoms.

**4.** A composition according to claim 1, wherein $R_1$ is alkyl of 6 to 18 carbon atoms, $-NR_6R_6$ or phenyl, and $R_2$ and $R_3$ and $R_5$ and $R_6$ are each independently of the other alkyl of 1 to 12 carbon atoms and $R_4$ is alkylene of 2 to 10 carbon atoms.

**5.** A composition according to claim 1, wherein $R_1$ is phenyl or $-NR_6R_6$, and $R_2$ and $R_3$ and $R_6$ and $R_6$ are each independently of the other alkyl of 1 to 8 carbon atoms.

**6.** A composition according to claim 1, wherein $R_2$ and $R_3$ are each independently of the other alkyl of 4 to

8 carbon atoms, and $R_4$ is alkylene of 1 to 10 carbon atoms or alkenylene of 2 to 10 carbon atoms.

**7.** A composition according to claim 1, wherein $R_2$ and $R_3$ are each independently of the other alkyl of 4 to 8 carbon atoms, and $R_4$ is alkylene of 3 to 8 carbon atoms.

**8.** A composition according to claim 2, wherein, when x is 1, $R_1$ is phenyl or $-NR_5R_6$ and $R_2$ and $R_3$ or $R_6$ and $R_6$ are identical and are each alkyl of 1 to 12 carbon atoms, or wherein, when x is 2, $R_4$ is alkylene of 3 to 8 carbon atoms and $R_2$ and $R_3$ are each alkyl of 3 to 10 carbon atoms.

**9.** A composition according to claim 1, which c) additionally comprises at least one aromatic amine and/or at least one alkali metal dithiophosphate.

**10.** A composition according to claim 9, wherein the aromatic amine is a compound of formula II or III or a mixture of such compounds

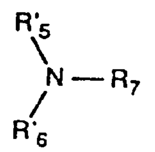 (II) 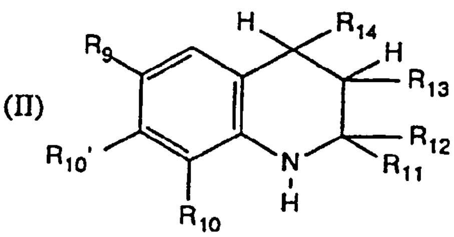 (III)

in which $R'_5$ is $C_1$-$C_{18}$alkyl, $C_7$-$C_9$phenylalkyl, $C_5$-$C_{12}$cycloalkyl, phenyl, $C_7$-$C_{18}$alkylphenyl, $C_7$-$C_{18}$alkoxyphenyl or naphthyl,
$R'_6$ is phenyl, $C_7$-$C_{18}$alkylphenyl, $C_7$-$C_{18}$alkoxyphenyl or naphthyl,
$R_7$ is hydrogen, $C_1$-$C_{12}$alkyl, benzyl, allyl, methallyl, phenyl or a group $-CH_2SR_6$, and
$R_8$ is $C_4$-$C_{18}$alkyl, $-CH_2COO(C_4$-$C_{18}$alkyl) or $-CH_2CH_2COO(C_4$-$C_{18}$alkyl) and
$R_9$ and $R_{10}$ are each independently of the other hydrogen, $C_1$-$C_{18}$alkyl or benzyl, and
$R_{10'}$ is hydrogen or $C_1$-$C_{12}$alkyl or, when taken together with $R_{10}$, forms a butadienediyl radical, and
$R_{11}$ and $R_{12}$ are each independently of the other $C_1$-$C_{18}$alkyl, phenyl or benzyl, or $R_{11}$ and $R_{12}$, together with the linking carbon atom, form a $C_5$-$C_{12}$spirocycloalkyl ring, and
$R_{13}$ is hydrogen or $C_1$-$C_{18}$alkyl, and
$R_{14}$ is $C_1$-$C_{18}$alkyl, or
$R_{13}$ and $R_{14}$, together with both linking carbon atoms, are a $C_5$-$C_{12}$cycloaliphatic radical.

**11.** A composition according to claim 10, wherein the compound of formula II is a technical mixture obtained by reacting diphenylamine with diisobutylene, said mixture comprising

| | |
|---|---|
| 1 to 5 % by weight of | a) diphenylamine |
| 8 to 18 % by weight of | b) 4-tert-butyldiphenylamine |
| 21 to 31 % by weight of | c) one or more of the compounds selected from |
| | i) 4-tert-octyldiphenylamine |
| | ii) 4,4′-di-tert-butyldiphenylamine |
| | iii) 2,4,4′-tris-tert-butyldiphenylamine, |
| 20 to 31 % by weight of | d) one or more of the compounds selected from |
| | i) 4-tert-butyl-4′-tert-octyldiphenylamine |
| | ii) 2,2′- or 2,4′-di-tert-octyldiphenylamine |
| | iii) 2,4-di-tert-butyl-4′-tert-octyldiphenylamine and |
| 15 to 29 % by weight of | e) the compound |
| | i) 4,4′-di-tert-octyldiphenylamine or of the compounds |
| | i) 4,4′-di-tert-octyldiphenylamine and |
| | ii) 2,4-di-tert-octyl-4′-tert-butyldiphenylamine. |

**12.** A composition according to claim 9, wherein the alkali metal dithiophosphate is a compound of formula IV

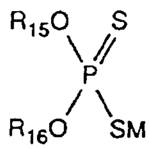

(IV)

in which $R_{15}$ and $R_{16}$ are each independently of the other $C_1$-$C_{24}$alkyl or $C_2$-$C_{12}$alkyl which is interrupted by -O-, -S- and/or -C(O)O-;
unsubstituted or $C_1$-$C_{12}$alkyl-substituted phenyl; $C_5$-$C_{12}$cycloalkylor $C_5$-$C_{12}$cycloalkyl which is substituted by $C_1$-$C_4$alkyl; or is $C_7$-$C_{13}$phenylalkyl or
$C_7$-$C_{13}$phenylalkyl which is interrupted in the alkyl radical by -O- or -S-, or $R_{15}$ and $R_{16}$, when taken together, are a dimethylene or trimethylene group or a dimethylene or trimethylene group which is substituted by $C_1$-$C_4$alkyl, and in which M is an alkali metal.

**13.** A method of stabilising a lubricant or hydraulic fluid against oxidative and/or thermal degradation, which comprises adding to the lubricant or hydraulic fluid at least one compound of formula I according to claim 1.

**14.** A compound of formula I as defined in claim 1, wherein x is 1, n is 2 and R and $R_1$ independently of each other are a group $-NR_5R_5$, where $R_5$ and $R_5$ are as defined in claim 1 and $R_2$ and $R_3$ are as defined in claim 1.

**15.** A compound according to claim 14, wherein $R_2$ and $R_3$ and $R_5$ and $R_5$ are each independently of the other $C_1$-$C_{12}$alkyl.

**Revendications**

**1.** Composition contenant
a) un lubrifiant ou un fluide hydraulique et
b) au moins un composé de formule générale I

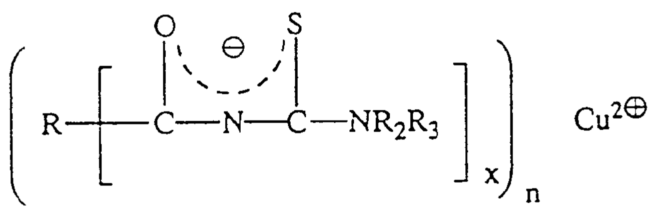

(I),

dans laquelle x = 1 ou 2 et, quand x = 1, n = 2 et R a la signification de $R_1$, ou, quand x = 2, n = 1 et R a la signification de $R_4$, et $R_1$ représente un alkyle de 1 à 25 atomes de carbone, un groupe cycloalkyle de 5 à 12 atomes de carbone non substitué ou substitué par alkyle en $C_1$-$C_8$, un alcényle de 2 à 18 atomes de carbone, un phényle, un naphtyle, un phénylalkyle en $C_7$-$C_{18}$, un alkylphényle en $C_7$-$C_{18}$ ou $-NR_5R_5$, et $R_2$ et $R_3$ sont identiques ou différents et représentent un reste alkyle de 1 à 18 atomes de carbone, phényle, naphtyle, phénylalkyle en $C_7$-$C_{18}$ ou alkylphényle en $C_7$-$C_{18}$, ou $R_2$ et $R_3$ forment ensemble, avec l'atome d'azote auquel ils sont liés, un noyau hétérocyclique de 5 à 7 chaînons qui peut éventuellement contenir en plus un atome d'azote ou d'oxygène, $R_4$ représente un alkylène de 1 à 12 atomes de carbone qui est éventuellement interrompu par au moins un groupe -O-, ou est un alcénylène de 2 à 12 atomes de carbone qui est éventuellement interrompu par au moins un groupe -O-, et $R_5$ et $R_6$ peuvent avoir les mêmes significations que $R_2$ et $R_3$.

**2.** Composition selon la revendication 1, dans laquelle $R_1$ représente un alkyle de 1 à 18 atomes de carbone, un groupe cycloalkyle non substitué de 5 à 8 atomes de carbone, un phényle, un alkylphényle en $C_7$-$C_{12}$, un phénylalkyle en $C_7$-$C_{12}$ ou $-NR_5R_6$, $R_2$ et $R_3$, respectivement $R_5$ et $R_5$, sont identiques ou différents et représentent chacun un alkyle en $C_1$-$C_{18}$ ou un phényle, ou bien $R_2$ et $R_3$, respectivement $R_5$ et $R_5$, forment ensemble, avec l'atome d'azote auquel ils sont liés, un groupe pipéridino, hexaméthylène-imino, pipérazino ou morpholino, et $R_4$ représente un alkylène de 1 à 12 atomes de carbone ou un alcénylène de 2 à

12 atomes de carbone.

3. Composition selon la revendication 1, dans laquelle $R_1$ représente un reste alkyle de 1 à 18 atomes de carbone, phényle, $-NR_5R_5$ ou benzyle, et $R_2$ et $R_3$, respectivement $R_5$ et $R_5$, sont identiques ou différents et représentent chacun un alkyle de 1 à 18 atomes de carbone.

4. Composition selon la revendication 1, dans laquelle $R_1$ représente un reste alkyle de 6 à 18 atomes de carbone, $-NR_5R_5$ ou phényle, $R_2$ et $R_3$, respectivement $R_5$ et $R_6$, représentent chacun, indépendamment les uns des autres, un alkyle de 1 à 12 atomes de carbone, et $R_4$ représente un alkylène de 2 à 10 atomes de carbone.

5. Composition selon la revendication 1, dans laquelle $R_1$ représente un phényle ou $-NR_5R_5$, et $R_2$ et $R_3$, respectivement $R_5$ et $R_5$, représentent chacun, indépendamment les uns des autres, un alkyle de 1 à 8 atomes de carbone.

6. Composition selon la revendication 1, dans laquelle $R_2$ et $R_3$ représentent, indépendamment l'un de l'autre, un alkyle de 4 à 8 atomes de carbone et $R_4$ est un reste alkylène de 1 à 10 atomes de carbone ou alcénylène de 2 à 10 atomes de carbone.

7. Composition selon la revendication 1, dans laquelle $R_2$ et $R_3$ représentent, indépendamment l'un de l'autre, un alkyle de 4 à 8 atomes de carbone et $R_4$ est un alkylène de 3 à 8 atomes de carbone.

8. Composition selon la revendication 2, dans laquelle, quand x = 1, $R_1$ est un reste phényle ou $-NR_5R_6$, et $R_2$ et $R_3$, respectivement $R_5$ et $R_6$, sont identiques et représentent chacun un alkyle de 1 à 12 atomes de carbone, ou, quand x = 2, $R_4$ est un alkylène de 3 à 8 atomes de carbone, et $R_2$ et $R_3$ sont chacun un alkyle de 3 à 10 atomes de carbone.

9. Composition selon la revendication 1, qui contient en plus c) au moins une amine aromatique et/ou au moins un dithiophosphate alcalin.

10. Composition selon la revendication 9, dans laquelle l'amine aromatique est un composé de formule II ou III ou un mélange de tels composés,

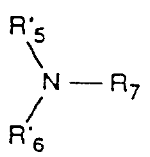 (II) 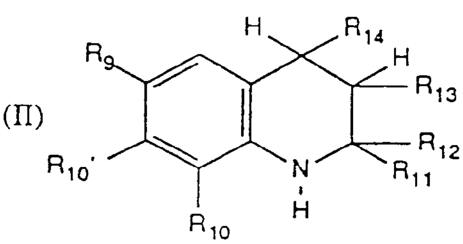 (III)

dans lesquelles $R_5'$ représente un reste alkyle en $C_1$-$C_{18}$, phénylalkyle en $C_7$-$C_9$, cycloalkyle en $C_5$-$C_{12}$, phényle, alkylphényle en $C_7$-$C_{18}$, alcoxyphényle en $C_7$-$C_{18}$ ou naphtyle,
$R_6'$ représente un reste phényle, alkylphényle en $C_7$-$C_{18}$, alcoxyphényle en $C_7$-$C_{18}$ ou naphtyle,
$R_7$ représente l'hydrogène, un reste alkyle en $C_1$-$C_{12}$, benzyle, allyle, méthallyle, phényle ou un groupe $-CH_2SR_5$, et
$R_8$ représente un reste alkyle en $C_4$-$C_{18}$, $CH_2COO$(alkyle en $C_4$-$C_{18}$) ou $-CH_2CH_2COO$(alkyle en $C_4$-$C_{18}$), et
$R_9$ et $R_{10}$ représentent indépendamment l'un de l'autre l'hydrogène ou un reste alkyle en $C_1$-$C_{18}$ ou benzyle, et
$R_{10}'$ représente l'hydrogène ou un alkyle en $C_1$-$C_{12}$, ou forme avec $R_{10}$ un reste butadiènediyle, et
$R_{11}$ et $R_{12}$ sont indépendamment l'un de l'autre un reste alkyle en $C_1$-$C_{18}$, phényle ou benzyle ou $R_{11}$ et $R_{12}$ forment ensemble avec l'atome de carbone auquel ils sont liés un noyau cycloalkyle en $C_5$-$C_{12}$ condensé en spiro, et
$R_{13}$ est l'hydrogène ou un alkyle en $C_1$-$C_{18}$ et
$R_{14}$ est un alkyle en $C_1$-$C_{18}$, ou
$R_{13}$ et $R_{14}$ forment ensemble, avec les deux atomes de carbone auxquels ils sont liés, un reste cycloaliphatique en $C_5$-$C_{12}$.

**11.** Composition selon la revendication 10, dans laquelle le composé de formule II est un mélange technique obtenu par réaction de diphénylamine avec du diisobutylène, contenant

| | |
|---|---|
| 1 à 5 % en masse | a) de diphénylamine |
| 8 à 18 % en masse | b) de 4-tert-butyldiphénylamine |
| 21 à 31 % en masse | c) d'un ou plusieurs des composés<br>i) 4-tert-octyldiphénylamine<br>ii) 4,4′-di-tert-butyldiphénylamine<br>iii) 2,4,4′-tris-tert-butyldiphénylamine |
| 20 à 31 % en masse | d) d'un ou plusieurs des composés<br>i) 4-tert-butyl-4′-tert-octyldiphénylamine<br>ii) 2,2′- ou 2,4′-di-tert-octyldiphénylamine<br>iii) 2,4-di-tert-butyl-4′-tert-octyldiphénylamine et |
| 15 à 29 % en masse | e) du composé<br>i) 4,4′-di-tert-octyldiphénylamine ou des composés<br>i) 4,4′-di-tert-octyldiphénylamine et<br>ii) 2,4-di-tert-octyl-4′-tert-butyldiphénylamine. |

**12.** Composition selon la revendication 9, dans laquelle le dithiophosphate alcalin est un composé de formule IV

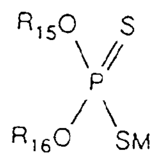

(IV)

dans laquelle $R_{15}$ et $R_{16}$ représentent indépendamment l'un de l'autre un alkyle en $C_1$-$C_{24}$ ou un alkyle en $C_2$-$C_{12}$ interrompu par -O-, -S- et/ou -C(O)O-; un phényle non substitué ou substitué par alkyle en $C_1$-$C_{12}$; un cycloalkyle en $C_5$-$C_{12}$ ou un cycloalkyle en $C_5$-$C_{12}$ substitué par alkyle en $C_1$-$C_4$; ou un phénylalkyle en $C_7$-$C_{13}$ ou un phénylalkyle en $C_7$-$C_{13}$ dont le reste alkyle est interrompu par -O- ou -S-, ou $R_{15}$ et $R_{16}$ représentent ensemble un groupe diméthylène ou triméthylène ou un groupe diméthylène ou triméthylène substitué par alkyle en $C_1$-$C_4$, et dans laquelle M représente un métal alcalin.

**13.** Procédé de stabilisation de lubrifiants ou de fluides hydrauliques contre la dégradation due à l'oxydation et/ou à la chaleur, caractérisé en ce que l'on ajoute au lubrifiant ou au fluide hydraulique au moins un composé de formule I selon la revendication 1.

**14.** Composés de formule I définie dans la revendication 1, dans laquelle x = 1, n = 2, et R ou $R_1$ représente un groupe $-NR_5R_5$, $R_5$ et $R_5$ étant définis comme dans la revendication 1, et $R_2$ et $R_3$ ont la signification donnée dans la revendication 1.

**15.** Composés selon la revendication 14, dans lesquels $R_2$ et $R_3$, ainsi que $R_5$ et $R_5$, représentent chacun, indépendamment les uns des autres, un alkyle en $C_1$-$C_{12}$.